(19) Europäisches Patentamt
European Patent Office
Office européen des brevets

(11) **EP 4 212 873 B1**

(12) **EUROPEAN PATENT SPECIFICATION**

(45) Date of publication and mention
of the grant of the patent:
**08.04.2026  Bulletin 2026/15**

(51) International Patent Classification (IPC):
**G01N 33/48** *(2006.01)*   **G01N 33/543** *(2006.01)*
**B01L 3/00** *(2006.01)*   **G01N 1/40** *(2006.01)*

(21) Application number: **21866476.1**

(22) Date of filing: **18.08.2021**

(52) Cooperative Patent Classification (CPC):
**G01N 1/4077; B01L 3/5021; B01L 3/5023;**
**G01N 1/4005; G01N 1/405;** B01L 2300/0681;
B01L 2300/069; B01L 2300/0825; G01N 2001/4088

(86) International application number:
**PCT/JP2021/030251**

(87) International publication number:
**WO 2022/054516 (17.03.2022 Gazette 2022/11)**

(54) **CONCENTRATION DEVICE, METHOD FOR CONCENTRATING SAMPLE SOLUTION, METHOD FOR TESTING SAMPLE SOLUTION, AND TEST KIT**

KONZENTRATIONSVORRICHTUNG, VERFAHREN ZUR KONZENTRATION EINER PROBENLÖSUNG, VERFAHREN ZUM TESTEN EINER PROBENLÖSUNG UND TESTKIT

DISPOSITIF DE CONCENTRATION, PROCÉDÉ DE CONCENTRATION D'UNE SOLUTION D'ÉCHANTILLON, PROCÉDÉ DE TEST D'UNE SOLUTION D'ÉCHANTILLON ET KIT DE TEST

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2020   JP 2020152941**

(43) Date of publication of application:
**19.07.2023   Bulletin 2023/29**

(73) Proprietor: **FUJIFILM Corporation**
**Tokyo 106-8620 (JP)**

(72) Inventors:
• **ABURAYA Yoshihiro**
**Ashigarakami-gun, Kanagawa 258-8538 (JP)**
• **YOSHIDA Junya**
**Ashigarakami-gun, Kanagawa 258-8538 (JP)**
• **ISHII Hiroyasu**
**Ashigarakami-gun, Kanagawa 258-8538 (JP)**
• **KATADA Junichi**
**Ashigarakami-gun, Kanagawa 258-8538 (JP)**

(74) Representative: **Dehns Germany Partnerschaft mbB**
**Theresienstraße 6-8**
**80333 München (DE)**

(56) References cited:
EP-A1- 3 845 900      WO-A1-02/38503
WO-A1-2020/045524      WO-A1-2020/045625
WO-A1-2020/089797      WO-A1-2020/108390
JP-A- 2000 072 822      JP-A- 2008 506 930
JP-A- 2013 543 110      US-A1- 2018 292 398
US-B2- 7 833 796

• WU XUNYI ET AL: "Synthesis and application of superabsorbent polymer microspheres for rapid concentration and quantification of microbial pathogens in ambient water", SEPARATION AND PURIFICATION TECHNOLOGY, ELSEVIER SCIENCE, AMSTERDAM, NL, vol. 239, 116540, 11 January 2020 (2020-01-11), pages 1 - 8, XP085995912, ISSN: 1383-5866, [retrieved on 20200111], DOI: 10.1016/J.SEPPUR.2020.116540

**EP 4 212 873 B1**

# EP 4 212 873 B1

**Description**

## BACKGROUND OF THE INVENTION

1. Field of the Invention

The present invention relates to a concentration device, a sample solution concentration method, a sample solution examination method, and an examination kit.

2. Description of the Related Art

In the related art, a technique of concentrating an aqueous solution containing a high-molecular-weight molecule contained in a biological fluid such as an antigen (hereinafter, also referred to as a "sample solution") with an ultrafiltration instrument is known (for example, JP1992-355339A (JP-H4-355339A)). Another technique of concentrating an aqueous solution is described in WO 2020/108390.

## SUMMARY OF THE INVENTION

Recently, there is a demand for a more sensitive method in an immunological examination method such as immunochromatography. Under such circumstances, the inventors of the present invention studied a method of concentrating a sample solution using an ultrafiltration instrument and carrying out an immunological examination using the concentrated sample solution. As a result, it was revealed that although the sensitivity is certainly improved by such a method, on the other hand, the concentration of the sample solution using the ultrafiltration instrument takes a long time and is not suitable for point of care testing (POCT).

In consideration of the above circumstances, an object of the present invention is to provide a concentration device that makes it possible to concentrate a sample solution in a short time, a sample solution concentration method using the concentration device to concentrate a sample solution, a sample solution concentration method using the sample solution examination method, and an examination kit including the concentration device and the detection device.

As a result of carrying out intensive studies to achieve the above-described object, the inventors of the present invention found that the above-described object can be achieved by the following configurations.

(1) A concentration device for concentrating a sample solution which is an aqueous solution containing a high-molecular-weight molecule contained in a biological fluid, the concentration device comprising:

a first container containing a super absorbent polymer; and
a second container,
in which a part of the first container is constituted of a discharge unit consisting of a porous membrane having a hole diameter of 0.05 $\mu$m or more and 10 $\mu$m or less, and wherein the porous membrane consists of a material having a surface energy of $24.10^{-3}$ N/m (24 dynes/ cm) or more and $75.10^{-3}$ N/m (75 dynes/cm) or less,
the super absorbent polymer is configured to absorb a solution contained in the sample solution injected into the first container to generate, in the first container, a sample solution concentrated solution which is a concentrated solution of the sample solution, and
the second container is configured to recover the sample solution concentrated solution discharged from the discharge unit.

(2) The concentration device according to (1), in which a material of the porous membrane is at least one selected from the group consisting of cellulose acetate, polyether sulfone, and glass fiber.
(3) The concentration device according to (1) or (2), in which the porous membrane is consisting of a material having a surface energy of $40.10^{-3}$ N/m (40 dynes/cm) or more and $75.10^{-3}$ N/m (75 dynes/cm) or less.
(4) The concentration device according to any one of (1) to (3), in which a swelling ratio of the super absorbent polymer is more than 0.2 g/g and less than 800 g/g.
(5) The concentration device according to any one of (1) to (4), in which the super absorbent polymer is a polyacrylic acid-based, polyacrylamide-based, cellulose-based, or polyethylene oxide-based polymer.
(6) The concentration device according to any one of (1) to (5), in which the high-molecular-weight molecule contained in the biological fluid is an antigen.
(7) The concentration device according to any one of (1) to (6), in which the sample solution is urine.
(8) The concentration device according to (7), in which a concentration of urea in the sample solution concentrated solution is 5-fold or less with respect to the concentration of the urea in the sample solution.

(9) A sample solution concentration method in which a sample solution which is an aqueous solution containing a high-molecular-weight molecule contained in a biological fluid is concentrated using the concentration device according to any one of (1) to (8), the sample solution concentration method comprising, in the following order:

a sample solution injection step of injecting the sample solution into the first container;
a water absorption step in which a solution contained in the sample solution injected into the first container is absorbed by a super absorbent polymer accommodated in the first container to generate, in the first container, a sample solution concentrated solution, which is a concentration solution of the sample solution;
a discharge step of applying an external force to the sample solution concentrated solution to discharge the sample solution concentrated solution from the discharge unit; and
a recovery step of recovering the sample solution concentrated solution discharged from the discharge unit, in the second container.

(10) The sample solution concentration method according to (9), in which the external force is a centrifugal force.
(11) The sample solution concentration method according to (10), in which the centrifugal force is applied under a condition of 4,000 to 10,000 rpm.
(12) A sample solution examination method in which a high-molecular-weight molecule contained in a biological fluid in a sample solution which is an aqueous solution containing the high-molecular-weight molecule contained in the biological fluid is detected, the sample solution examination method comprising, in the following order:

a concentration step of using the sample solution concentration method according to any one of (9) to (11) to obtain the sample solution concentrated solution; and
a detection step of detecting the high-molecular-weight molecule contained in the biological fluid in the obtained sample solution concentrated solution.

(13) The examination method according to (12),

in which the sample solution is an aqueous solution in which an antigen is containable,
the concentration step is a step of using the sample solution concentration method according to any one of (9) to (11) to concentrate an aqueous solution in which the antigen is containable, to obtain an antigen-concentrated solution, and
the detection step is a step of detecting the antigen in the antigen-concentrated solution by immunochromatography using an antigen-antibody reaction.

(14) An examination kit for detecting a high-molecular-weight molecule contained in a biological fluid in a sample solution which is an aqueous solution containing the high-molecular-weight molecule contained in the biological fluid, the examination kit comprising:

the concentration device according to any one of (1) to (8); and
a detection device that includes an examination strip having an examination region for detecting the high-molecular-weight molecule contained in the biological fluid, a first pot and a second pot in which a first amplification solution and a second amplification solution for amplifying an examination signal in the examination region are enclosed respectively, and a housing case encompassing the examination strip, the first pot, and the second pot.

As will be described below, according to the present invention, it is possible to provide a concentration device that makes it possible to concentrate a sample solution in a short time, a sample solution concentration method using the concentration device to concentrate a sample solution, a sample solution examination method using the sample solution concentration method, and an examination kit including the concentration device and the detection device.

## BRIEF DESCRIPTION OF THE DRAWINGS

Fig. 1 is a schematic cross-sectional view of a suitable aspect of the concentration device according to the present invention.
Fig. 2 is a schematic cross-sectional view of a mini-centrifugal filtration filter that is used in Examples.
Fig. 3A is a view illustrating a sample solution injection step among schematic cross-sectional views illustrating a suitable aspect of the concentration method according to the present invention in the order of steps.

Fig. 3B is a view illustrating a water absorption step among schematic cross-sectional views illustrating a suitable aspect of the concentration method according to the present invention in the order of steps.

Fig. 3C is a view illustrating a discharge step among schematic cross-sectional views illustrating a suitable aspect of the concentration method according to the present invention in the order of steps.

Fig. 3D is a view illustrating a recovery step among schematic cross-sectional views illustrating a suitable aspect of the concentration method according to the present invention in the order of steps.

Fig. 4 is a schematic view of one aspect of an insoluble carrier that is used in a detection step of an examination method according to the present invention.

Fig. 5 is a perspective view illustrating an aspect of one embodiment of an immunochromatographic kit.

Fig. 6 is an exploded schematic perspective view illustrating an aspect of one embodiment of the immunochromatographic kit.

Fig. 7 is a schematic side view illustrating a positional relationship between an examination strip and a first and second pots.

Fig. 8 is a perspective view of a first convex deformation part provided in an upper case of the immunochromatographic kit illustrated in Fig. 5.

Fig. 9 is end views of a cut part cut along a V-V' line before and after the deformation of a first convex deformation part illustrated in Fig. 8.

Fig. 10 is a perspective view of a second convex deformation part provided in an upper case of the immunochromatographic kit illustrated in Fig. 5.

Fig. 11 is end views of a cut part cut along a VII-VII' line before and after the deformation of a second convex deformation part illustrated in Fig. 10.

Fig. 12 is end views of a cut part before and after the deformation of a convex deformation part in a design modification example.

Fig. 13 is a perspective view of Amicon Ultra that is used in Comparative Example A4 and Comparative Example B4.

## DESCRIPTION OF THE PREFERRED EMBODIMENTS

Hereinafter, a concentration device according to the embodiment of the present invention, a sample solution concentration method according to the embodiment of the present invention, a sample solution examination method according to the embodiment of the present invention, and an examination kit according to the embodiment of the present invention will be described.

In the present specification, the numerical value range indicated by using "to" means a range including the numerical values before and after "to" as the lower limit value and the upper limit value, respectively.

In addition, in the present specification, one kind of each component may be used alone, or two or more kinds thereof may be used in combination. In a case where two or more kinds of each component are used in combination, a content of the component indicates a total content unless otherwise specified.

In addition, in the present specification, "the concentration time shortens", "the concentration rate increases", "the detection sensitivity increases", and "the S/N ratio (the signal/noise ratio) increases" are also simply referred to that the effect and the like of the present invention are excellent.

[1] Concentration device

The concentration device according to the embodiment of the present invention is

a concentration device for concentrating a sample solution which is an aqueous solution containing a high-molecular-weight molecule contained in a biological fluid, the concentration device including;
a first container containing a super absorbent polymer and a second container,
in which a part of the first container is constituted of a discharge unit consisting of a porous membrane having a hole diameter of 0.05 $\mu$m or more and 10 $\mu$m or less,
the super absorbent polymer absorbs a solution (water or a low-molecular-weight molecule) contained in the sample solution injected into the first container to generate, in the first container, a sample solution concentrated solution which is a concentrated solution of the sample solution, and
the second container recovers the sample solution concentrated solution discharged from the discharge unit.

First, a suitable aspect of the concentration device according to the embodiment of the present invention will be described with reference to the drawings.

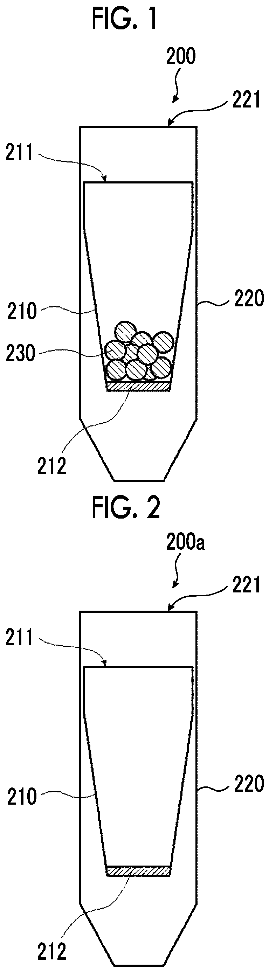

Fig. 1 is a schematic cross-sectional view of a suitable aspect of the concentration device according to the embodiment of the present invention.

As illustrated in Fig. 1, a concentration device 200 includes a first container 210 that accommodates a super absorbent polymer 230, and a second container 220. A bottom part of the first container 210 is constituted of a discharge unit 212 consisting of a porous membrane having a hole diameter of 0.05 $\mu$m or more and 10 $\mu$m or less. The first container 210 has an opening portion 211. In addition, the second container 220 has an opening portion 221. In addition, the first container 210 is accommodated in the second container 220, and the first container 210 is fixed in the second container 220 so that a space is generated between the discharge unit 212, which is the bottom part of the first container 210, and the bottom surface of the second container.

Hereinafter, each member constituting the concentration device according to the embodiment of the present invention will be described.

It is noted that the sample solution and the like will be described later in the sample solution concentration method according to the embodiment of the present invention.

[First container]

As described above, a part of the first container is constituted of the discharge unit consisting of a porous membrane having a hole diameter of 0.05 $\mu$m or more and 10 $\mu$m or less. In addition, as described above, the first container accommodates the super absorbent polymer.

The shape of the first container is not particularly limited; however, it is preferably tubular (cylindrical) and more preferably cylindrical.

The material of the first container is not particularly limited; however, it is preferably a thermoplastic resin since thermoplastic resin can be subjected to injection molding, is inexpensive, and can be produced on a large scale. Specifically, it is preferably polypropylene, acryl, polyacetal, polyamide, polyethylene, polyethylene terephthalate, polycarbonate, polystyrene, polyphenylene sulfide, polybutylene terephthalate, polyvinyl chloride, an acrylonitrile-butadiene-styrene copolymer resin (an ABS resin), or an acrylonitrile-styrene copolymer resin (an AS resin) since this has a certain degree of hardness.

[Discharge unit]

As described above, a part of the first container is constituted of the discharge unit consisting of a porous membrane having a hole diameter of 0.05 $\mu$m or more and 10 $\mu$m or less.

The position of the discharge unit is not particularly limited; however, it is preferable that the bottom part of the first container is constituted of the discharge unit.

<Porous membrane>

The porous membrane constituting the discharge unit is a porous membrane having a hole diameter of 0.05 $\mu$m or more and 10 $\mu$m or less. Since the hole diameter of the porous membrane is 10 $\mu$m or less, the injected sample solution is not discharged from the discharge unit under atmospheric pressure and held in the first container in a case where the sample solution is injected into the first container.

(Pore diameter)

The upper limit of the hole diameter of the porous membrane is preferably 5 or less, more preferably 3 $\mu$m or less, and still more preferably 1 $\mu$m or less, due to the reason that the effects and the like of the present invention are more excellent.

The lower limit of the hole diameter of the porous membrane is preferably 0.1 $\mu$m or more, more preferably 0.3 $\mu$m or more, and still more preferably 0.5 $\mu$m or more, due to the reason that the effects and the like of the present invention are more excellent.

The measurement of the hole diameter can be carried out by a known method such as an optical microscopy method, mercury porosimetry, or a BET method; however, in the present invention, the hole diameter shall be measured according to a bubble point method (based on JIS K 3832) using a Perm Poromometer (manufactured by Porous Materials Inc.).

(Material)

The material of the porous membrane is not particularly limited. However, due to the reason that the effect and the like of the present invention are more excellent, it is preferably at least one selected from the group consisting of cellulose acetate (CA), polyether sulfone (PES), polytetrafluoroethylene (PTFE), glass fiber (GF), nylon, polyvinylidene fluoride (PVDF), and polyolefin, it is more preferably at least one selected from the group consisting of cellulose acetate (CA), polyether sulfone (PES), hydrophilic polytetrafluoroethylene (hydrophilic PTFE), glass fiber (GF), nylon, polyvinylidene fluoride (PVDF), and polyolefin, it is still preferably at least one selected from the group consisting of cellulose acetate (CA), polyether sulfone (PES), glass fiber (GF), nylon, polyvinylidene fluoride (PVDF), and polyolefin, and it is particularly preferably at least one selected from the group consisting of cellulose acetate (CA), polyether sulfone (PES), and glass fiber (GF), and polyolefin.

It is noted that in the present specification, the hydrophilic PTFE is a PTFE having a surface energy of $25.10^{-3}$ N/m (25 dynes/cm) or more, where the surface energy will be described later, and the hydrophobic PTFE is a PTFE having a surface energy of less than $25.10^{-3}$ N/m (25 dynes/ cm), where the surface energy will be described later.

(Surface energy)

Due to the reason that the effect and the like of the present invention are more excellent, the porous membrane according to the present invention consists of a material having a surface energy of $24.10^{-3}$ N/m (24 dynes/cm) or more and $75.10^{-3}$ N/m (75 dynes/cm) or less, and more preferably consists of a material having a surface energy of $40.10^{-3}$ N/m (40 dynes/cm) or more and $75.10^{-3}$ N/m (75 dynes/cm) or less.

In the measurement of the surface energy, a value obtained by measuring the contact angle of a liquid droplet formed in a case where each of water, diiodomethane (methylene iodide), and n-hexadecane is dropped on a film made of a polymer material in an environment of 20°C and 50% RH and being calculated according to the following method described in Reference Document 1 shall be used.

In a case of assuming that the surface energy consists of a dispersion component $\gamma^d$, a polar component $\gamma^p$, and a hydrogen-bonding component $\gamma^h$, the surface energy can be expressed as

$$\gamma = \gamma^d + \gamma^p + \gamma^h \,(... \text{ Expression (1)})$$

In a case where the surface energy of the liquid is denoted by $\gamma_L$, the surface energy of the solid is denoted by $\gamma_S$, and the measured contact angle is denoted by $\theta$, and the numerical values described in Tables 1 to 3 of Reference Document 1 are used as the $\gamma^d$, $\gamma^p$, and $\gamma^h$ values of water, diiodomethane, and n-hexadecane, the surface energy is calculated by solving simultaneous equations of $\gamma_s{}^d$, $\gamma_s{}^p$, and $\gamma_s{}^h$ using Expression (2).

$$\gamma_L(1 + \cos\theta) = 2(\gamma_S{}^d\gamma_L{}^d)^{1/2} + 2(\gamma_S{}^p\gamma_L{}^p)^{1/2} + 2(\gamma_S{}^h\gamma_L{}^h)^{1/2} \,(... \text{ Expression (2)})$$

(Reference Document 1: Yasuaki Kitazaki et al., Journal of the Adhesion Society of Japan, Vol.8, No.3, 1972, pp.131-141)

[Super absorbent polymer]

The super absorbent polymer (SAP) is not particularly limited; however, due to the reason that the effects and the like of the present invention are more excellent, it is preferably a polyacrylic acid-based, polyacrylamide-based, cellulose-based, or polyethylene oxide-based polymer.

<Swelling ratio>

The swelling ratio of the super absorbent polymer is not particularly limited; however, due to the reason that the effect and the like of the present invention are more excellent, it is preferably more than 0.2 g/g and less than 800 g/g, more preferably 1.0 g/g or more and 600 g/g or less, still more preferably 10 g/g or more and 500 g/g or less, and particularly preferably 20 g/g or more and 100 g/g or less.

Here, the swelling ratio is a value defined as "a mass (g) of water retained by 1 g of a super absorbent polymer".

(Method of measuring swelling ratio)

A mass of a super absorbent polymer stored for 10 days at 25°C and 5% of relative humidity (RH) is measured, and immediately after the measurement, the super absorbent polymer is immersed in a large amount of distilled water. After 120 minutes, the super absorbent polymer is taken out, the water on the surface thereof is removed, the mass thereof is measured again, and the swelling ratio thereof is measured using the following expression.

{(Mass (g) after water absorption -initial mass (g) before water absorption)/initial mass (g) before water absorption}

The method of adjusting the swelling ratio to the above-described specific range is not particularly limited; however, examples thereof include changing the kind of the polymer, changing the molecular weight of the polymer, changing the degree of crosslinking of the polymer, and changing the particle diameter of the polymer.

<Water absorption rate>

The water absorption rate of the super absorbent polymer is not particularly limited; however, due to the reason that the effect and the like of the present invention are more excellent, it is preferably 0.01 g/min or more and 40 g/min or less per 1 g of super absorbent polymer, and more preferably 0.02 g/min or more and 40 g/min or less per 1 g of super absorbent polymer.

The water absorption rate is measured as follows.

A mass (a weight $M_0$, unit: g) of a super absorbent polymer stored for 10 days at 25°C and 5% of relative humidity (RH) is measured, and immediately after the measurement, the super absorbent polymer is immersed in a large amount of distilled water. After 10 minutes, the super absorbent polymer is taken out, the water on the surface thereof is removed, and the mass thereof (a mass $M_{10}$) is measured. Immediately after the mass measurement, the super absorbent polymer is immersed again in a large amount of distilled water. After 10 minutes, the super absorbent polymer is taken out, the water on the surface thereof is removed, and the mass thereof (a mass $M_{20}$) is measured again. Immediately after the measurement of mass $M_{20}$, the super absorbent polymer is immersed again in a large amount of distilled water. After 10 minutes, the super absorbent polymer is taken out, the water on the surface thereof is removed, and the weight thereof (a mass $M_{30}$) is measured again.

The water absorption amount is defined as follows.

Water absorption amount for 10 minutes: $\Delta M10 = (M_{10} - M_0)/M_0$ Water absorption amount for 20 minutes: $\Delta M20 = (M_{20} - M_0)/M_0$ Water absorption amount for 30 minutes:

$$\Delta M30 = (M_{30} - M_0)/M_0$$

Using the water absorption amount defined as described above, the water absorption rate is calculated as follows.

Three points are plotted on the X-Y plane as the horizontal axis of time (x = 10, 20, 30, unit: minute) and the vertical axis of water absorption amount (y = $\Delta M10$, $\Delta M20$, $\Delta M30$, unit: g (water)/g (polymer amount)), to obtain a linear approximate expression of the water absorption amount with respect to the time by using the least squares method, and the slope of the linear approximate expression is defined as the water absorption rate per unit time (minute).

<Particle diameter>

The super absorbent polymer preferably has a particle shape, and the particle diameter in such a case is preferably 10 mm or less, more preferably 8 mm or less, and still more preferably 5 mm or less, due to the reason that the effect and the like of the present invention are more excellent. The lower limit of the particle diameter of the super absorbent polymer is preferably 0.001 mm or more, more preferably 0.005 mm or more, and still more preferably 0.01 mm or more, due to the reason that the effect and the like of the present invention are more excellent.

In addition, the ratio (the particle diameter/the hole diameter) of the particle diameter to the hole diameter of the above-described porous membrane is preferably 1 to 1,000 and preferably 10 to 100, due to the reason that the effect and the like of the present invention are more excellent.

Here, the particle diameter can be determined as an arithmetic average value obtained by measuring the diameters of 50 particulate polymers with an optical microscope.

[Second container]

The second container is a container for recovering the sample solution concentrated solution discharged from the discharge unit of the first container described above.

The second container preferably encompasses at least the discharge unit of the first container described above and more preferably encompasses the whole of the first container described above.

The suitable aspects of the shape and the material of the second container are the same as those of the first container.

[2] Sample solution concentration method

The sample solution concentration method according to the embodiment of the present invention (hereinafter, also referred to as "the concentration method according to the invention") is

a sample solution concentration method of concentrating a sample solution which is an aqueous solution containing a high-molecular-weight molecule contained in a biological fluid, by using the above-described concentration device, the sample solution concentration method including, in the following order;
a sample solution injection step of injecting the sample solution into the first container,
a water absorption step in which a solution (water or a low-molecular-weight molecule) contained in the sample solution injected into the first container is absorbed by the super absorbent polymer accommodated in the first container, and the sample solution concentrated solution which is a concentrated solution of the sample solution is generated in the first container,
a discharge step of applying an external force to the sample solution concentrated solution to discharge the sample solution concentrated solution from the discharge unit, and
a recovery step of recovering the sample solution concentrated solution discharged from the discharge unit, in the second container.

First, a suitable aspect of the concentration method according to the embodiment of the present invention will be described with reference to the drawings.

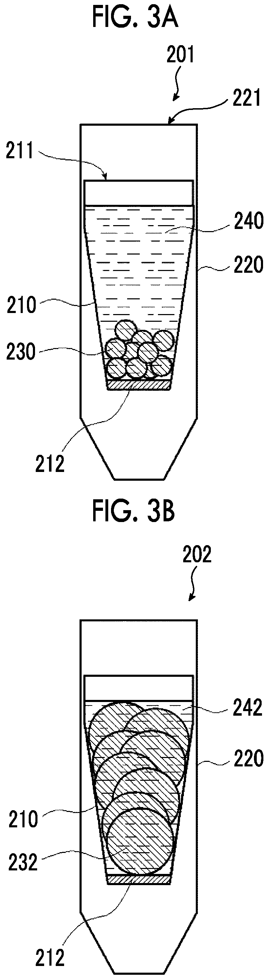

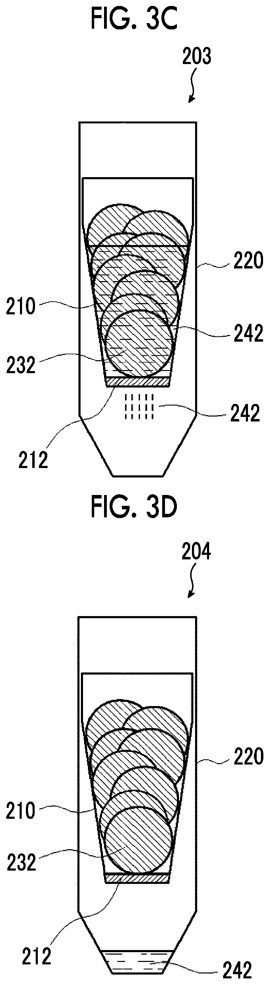

Fig. 3 (Fig. 3A to Fig. 3E) are schematic cross-sectional views illustrating a suitable aspect of the concentration method according to the embodiment of the present invention in the order of steps.
First, in the sample solution injection step, the sample solution 240 is injected into the first container 210 from the opening portion 221 of the second container 220 and the opening portion 211 of the first container 210 in the concentration device 200 of Fig. 1 (Fig. 3A). The concentration device 200 is as described above. Here, a bottom part of the first container 210 is constituted of a discharge unit 212 consisting of a porous membrane having a hole diameter of 0.05 $\mu$m or more and 10 $\mu$m or less. However, since the hole diameter of the porous membrane is 10 $\mu$m or less as described above, the injected sample solution 240 is held in the first container 210 without being discharged from the discharge unit 212.
Then, in the water absorption step, the solution (the water or the low-molecular-weight molecule) contained in the sample solution 240 is absorbed by the super absorbent polymer 230, and a sample solution concentrated solution 242, which is a concentrated solution of the sample solution 240, is generated in the first container 210 (the super absorbent polymer 230 becomes a swollen super absorbent polymer 232) (Fig. 3B).
Next, in the discharge step, the concentration device 202 after the water absorption step is placed in a small tabletop centrifuge, and a centrifugal force is applied to the sample solution concentrated solution 242 in the direction from the top to the bottom (the direction of the discharge unit) of Fig. 3C, whereby the sample solution concentrated solution 242 is discharged from the discharge unit 212 (Fig. 3C). It is noted that, as described above, since the hole diameter of the porous membrane is 0.05 $\mu$m or more, the discharge can be carried out even with a small tabletop centrifuge (for example, 4,000 to 10,000 revolutions per minute (rpm)) (in a case of such an ultrafiltration instrument as described in JP2013-527225A, a large centrifuge is required). In addition, the number of times of centrifugation is only required to be one time (in a case of such an ultrafiltration instrument as described in JP2013-527225A, the number of times of centrifugation is required to be two times as shown in Comparative Example A4 and Comparative Example B4 described later). As described above, the above-described method is extremely simple as compared with such an ultrafiltration instrument as described in JP2013-527225A (a small tabletop centrifuge can be used, and the number of times of centrifugation is only one time).
Then, in the recovery step, the sample solution concentrated solution 242 discharged from the discharge unit 212 is recovered in the second container 220 (Fig. 3D). In this way, a sample solution concentrated solution can be obtained.
Hereinafter, each of the steps will be described.

[Sample solution injection step]

The sample solution injection step is a step of injecting the sample solution into the above-described first container of the concentration device according to the embodiment of the present invention. By the sample solution injection step, the sample solution is injected into the first container, and the sample solution is mixed with the super absorbent polymer accommodated in the first container. Here, a part of the first container is constituted of the discharge unit consisting of a porous membrane having a hole diameter of 0.05 $\mu$m or more and 10 $\mu$m or less. However, as described above, since the hole diameter of the porous membrane is 10 $\mu$m or less, the injected sample solution is held in the first container without being discharged from the discharge unit.

[Concentration device]

The concentration device according to the embodiment of the present invention is as described above.

[Sample solution]

The sample solution is an aqueous solution containing a high-molecular-weight molecule contained in a biological fluid.
Specific examples of the sample solution include a biological specimen, particularly a biological specimen of animal origin (particularly, of human origin) such as a body fluid (for example, blood, serum, plasma, spinal fluid, tear fluid, sweat, urine, pus, nasal discharge, or sputum), and a mouthwash.
The sample solution is preferably urine due to the reason that the effects and the like of the present invention are more excellent.

<High-molecular-weight molecule contained in biological fluid>

The high-molecular-weight molecule (particularly the antigen) contained in the biological fluid is, for example, a high-molecular-weight molecule that is useful mainly for determining a disease, and examples thereof include a fungus, a bacterium (for example, tubercle bacillus or lipoarabinomannan (LAM) included in the tubercle bacillus), bacteria, a virus (for example, an influenza virus), and a nuclear protein thereof, which are detected in biological fluids. LAM is a major antigen in tuberculosis and a glycolipid which is a major constitutional component of the cell membrane and the cell wall.
Due to the reason that the effect and the like of the present invention are more excellent, the high-molecular-weight molecule contained in the biological fluid is preferably an antigen, more preferably a virus (particularly, an influenza virus) or LAM, and still more preferably LAM.
The concentration of the high-molecular-weight molecule contained in the biological fluid in the sample solution is not particularly limited; however, it is preferably 0.0001 ng/mL or more and 1 mg/mL or less, more preferably 0.001 ng/mL or more and 1 µg/mL or less, and still more preferably 0.01 ng/mL or more and 1 ng/mL or less, due to the reason that the effect and the like of the present invention are more excellent.

<Pretreatment of sample solution>

Regarding the sample solution, it is possible to use a sample solution as it is or in a form of an extraction solution obtained by extracting an antigen using an appropriate solvent for extraction, in a form of a diluent solution obtained by diluting an extraction solution with an appropriate diluent, or in a form in which an extraction solution has been concentrated by an appropriate method.
As the solvent for extraction, it is possible to use a solvent (for example, water, physiological saline, and a buffer solution) that is used in a general immunological analysis method, or a water-miscible organic solvent with which a direct antigen-antibody reaction can be carried out by being diluted with such a solvent.

[Proportion of super absorbent polymer with respect to sample solution]

The proportion of the super absorbent polymer with respect to the sample solution is not particularly limited; however, it is preferably 0.01 to 100 g and more preferably 0.1 to 50 g with respect to 1 mL of the sample solution due to the reason that the effect and the like of the present invention are more excellent.

[Water absorption step]

The water absorption step is a step in which a solution (water or a low-molecular-weight molecule) contained in the sample solution injected into the first container is absorbed by the super absorbent polymer accommodated in the first container, and the sample solution concentrated solution which is a concentrated solution of the sample solution is generated in the first container.
In a case where the sample solution and the super absorbent polymer are mixed, the solution (the water or the low-molecular-weight molecule) in the sample solution is incorporated into the super absorbent polymer, whereas a high-molecular-weight molecule (for example, an antigen) contained in the biological fluid in the sample solution is hardly incorporated into the super absorbent polymer since the antigen has a certain degree of hydrodynamic radius and thus the network structure on the surface of the super absorbent polymer exhibits a sieving effect. As a result, the high-molecular-weight molecule (for example, an antigen) contained in the biological fluid in the sample solution is concentrated.
In a case where the sample solution is urine, the concentration of urea in the sample solution concentrated solution is preferably 5-fold or less with respect to the concentration of urea in the sample solution due to the reason that the effect and the like of the present invention are more excellent.

Examples of the water absorption step include a method of allowing the device according to the embodiment of the present invention, after the above-described sample solution injection step, to stand. The standing time is not particularly limited; however, it is preferably 1 to 30 minutes and more preferably 1 to 10 minutes due to the reason that the effect and the like of the present invention are more excellent.

[Discharge step]

The discharge step is a step of applying an external force to the sample solution concentrated solution to discharge the sample solution concentrated solution from the discharge unit.

In a case where an external force is applied to the sample solution concentrated solution, the sample solution concentrated solution is pressed against the discharge unit, and the sample solution concentrated solution is discharged from the discharge unit.

The external force is preferably a centrifugal force due to the reason that the effects and the like of the present invention are more excellent.

The centrifugal force is preferably applied by using a small (for example, a size of 50 $cm^3$ or less) tabletop centrifuge (a small tabletop centrifuge) due to the reason that the effect and the like of the present invention are more excellent.

The centrifugal force is preferably applied under the condition (the rotation speed) of 4,000 to 10,000 rotations/minute due to the reason that the effect and the like of the present invention are more excellent.

[Recovery step]

The recovery step is a step of recovering the sample solution concentrated solution discharged from the discharge unit, in the second container.

In this way, a sample solution concentrated solution can be obtained.

[3] Sample solution examination method

The sample solution examination method according to the embodiment of the present invention (hereinafter, also referred to as "the examination method according to the invention") is

a sample solution examination method in which a high-molecular-weight molecule contained in a biological fluid in a sample solution which is an aqueous solution containing the high-molecular-weight molecule contained in the biological fluid detected, the sample solution examination method comprising, in the following order;
a concentration step of using the above-described sample solution concentration method according to the embodiment of the present invention to obtain the sample solution concentrated solution; and
a detection step of detecting the high-molecular-weight molecule contained in the biological fluid in the obtained sample solution concentrated solution.

In the examination method according to the embodiment of the present invention, since the high-molecular-weight molecule contained in the biological fluid is detected using the sample solution concentrated solution obtained by the above-described concentration method according to the embodiment of the present invention, the high detection sensitivity can be obtained.

[Concentration step]

The method of obtaining the sample solution concentrated solution using the concentration method according to the embodiment of the present invention is as described above.

[Detection step]

The detection step is a step of detecting the high-molecular-weight molecule contained in the biological fluid in the obtained sample solution concentrated solution.

Due to the reason that the effect and the like of the present invention are more excellent, the detection step is preferably a method using an antigen-antibody reaction, and examples thereof include an enzyme-linked immuno-sorbent assay (EIA), a solid phase enzyme-linked immuno-sorbent assay (ELISA), a radioimmunoassay (RIA), a fluorescent immunoassay (FIA), a Western blot method, and immunochromatography. Among the above, immunochromatography is preferable due to the reason that the effect and the like of the present invention are more excellent.

[Suitable aspect]

Due to the reason that the effect and the like of the present invention are more excellent, the examination method according to the embodiment of the present invention is preferably an examination method in which

the sample solution is an aqueous solution in which an antigen (a high-molecular-weight molecule contained in a biological fluid) is containable,
the concentration step is a step of using the above-described concentration method according to the embodiment of the present invention to concentrate an aqueous solution in which the antigen is containable, thereby obtaining an antigen-concentrated solution (a sample solution concentrated solution), and
the detection step is a step of detecting the antigen in the antigen-concentrated solution by immunochromatography using an antigen-antibody reaction.

Here, due to the reason that the effect and the like of the present invention are more excellent, the detection step preferably includes;

a spreading step of spreading gold particle composite bodies on an insoluble carrier having a reaction site at which a second binding substance capable of binding to an antigen in the antigen-concentrated solution has been immobilized, in a state where the gold particle composite bodies which are composite bodies of the antigen and modified gold particles which are gold particles modified with a first binding substance capable of binding to the antigen are formed, and
a capturing step of capturing the gold particle composite bodies at the reaction site of the insoluble carrier.

Due to the reason that the effect and the like of the present invention are more excellent, the detection step preferably further includes
a silver amplification step of silver-amplifying the gold particle composite body captured in the capturing step.
Here, due to the reason that the effect and the like of the present invention are more excellent, it is preferable that at least one of the first binding substance or the second binding substance is preferably a monoclonal antibody, and it is more preferable that both of the first binding substance and the second binding substance are a monoclonal antibody.
It is noted that the sample solution may contain impurities such as a low-molecular-weight component and a salt. For example, in a case where the sample solution is urine, it contains impurities such as urea. From the studies by inventors of the present invention, it was found that in a case where these impurities are concentrated together with a high-molecular-weight molecule contained in the biological fluid, the antigen-antibody reaction may be inhibited and the detection sensitivity is decreased. That is, it is known that the effect of improving the detection sensitivity by concentration may not be sufficiently obtained.
Therefore, in the above-described concentration device according to the embodiment of the present invention which is used in the above-described concentration step, the swelling ratio of the super absorbent polymer is preferably in the above-described preferred range. Within the above range, these impurities are incorporated into the super absorbent polymer together with water, the above-described decrease in detection sensitivity hardly occurs, and as a result, it is conceived that extremely high detection sensitivity is achieved.
Hereinafter, each step included in the above-described suitable aspect (hereinafter, also referred to as "the method according to the embodiment of the present invention") will be described.

[Spreading step]

The spreading step is a step of spreading gold particle composite bodies on an insoluble carrier having a reaction site at which a second binding substance capable of binding to an antigen in the antigen-concentrated solution obtained in the above-described concentration step has been immobilized, in a state where the gold particle composite body which is a composite body of the antigen and the modified gold particle which is a gold particle modified with a first binding substance capable of binding to the antigen is formed.

<Gold particle composite body>

As described above, in the spreading step, first, the gold particle composite body which is a composite body of the antigen in the antigen-concentrated solution obtained in the above-described concentration step and a modified gold particle which is a gold particle modified with a first binding substance capable of binding to the antigen is formed.

(Modified gold particle)

The modified gold particle is a gold particle modified with the first binding substance capable of binding to an antigen.

(1) Gold particle

The gold particle is not particularly limited; however, it is preferably a gold colloid particle due to the reason that the effect and the like of the present invention are more excellent.
In a case where the method according to the embodiment of the present invention includes a silver amplification step described later, the gold particle acts as a catalyst that reduces silver ions in the silver amplification step.
The particle diameter of the gold particles is preferably 500 nm or less, more preferably 300 nm or less, still more preferably 200 nm or less, and particularly preferably 100 nm or less, due to the reason that the effect and the like of the present invention are more excellent.
The lower limit of the particle diameter of the gold particles is not particularly limited; however, it is preferably 1 nm or more, more preferably 2 nm or more, and still more preferably 5 nm or more, due to the reason that the effect and the like of the present invention are more excellent.
The particle diameter can be measured with a commercially available particle diameter distribution meter or the like. As a method of measuring the particle diameter distribution, optical microscopy, confocal laser microscopy, electron microscopy, atomic force microscopy, static light scattering method, laser diffraction method, dynamic light scattering method, centrifugal sedimentation method, electric pulse measurement method, chromatography method, ultrasonic attenuation method, and the like are known, and apparatuses corresponding to the respective principles are commercially available. As the method of measuring a particle diameter, a dynamic light scattering method can be preferably used due to the particle diameter range and the ease of measurement. Examples of the commercially available measuring device using dynamic light scattering include NANOTRAC UPA (Nikkiso Co., Ltd.), a dynamic light scattering type particle diameter distribution measuring device LB-550 (HORIBA, Ltd.), and a Fiber-Optics Particle Analyzer FPAR-1000 (Otsuka Electronics Co., Ltd.). In the present invention, the average particle diameter is obtained as a value of a median diameter (d = 50) measured at a measurement temperature of 25°C.

(2) First binding substance

The first binding substance is not particularly limited as long as it is capable of binding to the above antigen; however, due to the reason that the effect and the like of the present invention are more excellent, it is preferably a protein, more preferably an antibody (for example, a polyclonal antibody or a monoclonal antibody), and from the viewpoint of achieving higher detection sensitivity, it is still more preferably a monoclonal antibody.
The above antibody is not particularly limited. However, it is possible to use, for example, an antiserum prepared from a serum of an animal immunized with an antigen, or an immunoglobulin fraction purified from an antiserum. In addition, it is possible to use a monoclonal antibody obtained by cell fusion using spleen cells of an animal immunized with an antigen, or a fragment thereof [for example, F(ab')2, Fab, Fab', or Fv]. The preparation of these antibodies can be carried out by a conventional method.
In a case where the antigen is LAM, examples of the first binding substance include the A194-01 antibody described in WO2017/139153A.
In a case where the antigen is LAM, other examples of the first binding substance include the antibody having a sequence described as MoAb1 in paragraph No. [0080] of WO2013/129634A.

(3) Method of manufacturing modified gold particle

The method of manufacturing the modified gold particle is not particularly limited, and a known method can be used. Examples thereof include a chemical bonding method such as a method in which an SH group is introduced into an antibody, and the fact that gold and an SH group are chemically bonded is utilized so that the SH bond of the antibody is cleaved to generate an Au-S bond on the Au surface when the antibody approaches gold particles, whereby the antibody is immobilized.

<Insoluble carrier>

The above-described insoluble carrier is an insoluble carrier having a reaction site (a test line) at which a second binding substance capable of binding to the antigen is immobilized. The insoluble carrier may have a plurality of test lines depending on the kinds of antigens (for example, a test line for an influenza A type virus and a test line for an influenza B type virus). In addition, the insoluble carrier may have a control line on the downstream side of the test line in order to check the spreading of the gold particle composite bodies. Further, in a case where a reducing agent solution is used in the silver amplification step described later, a coloring reagent immobilization line may be provided on the downstream side of the test line in order to detect the reducing agent solution.

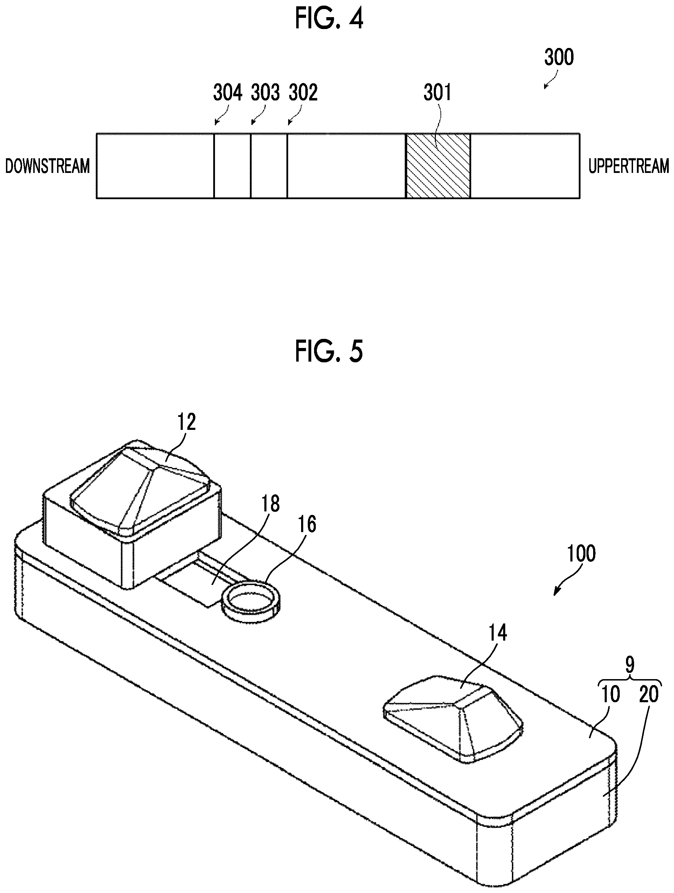

Examples of the specific aspect of the insoluble carrier include a nitrocellulose membrane 300 as illustrated in Fig. 4, which has from the upstream side; a gold colloid holding pad 301, a test line 302, a control line 303, and a coloring reagent immobilization line 304. Here, the gold colloid holding pad 301 is a pad that holds gold particles (modified gold particles) modified with the first binding substance, the test line 302 is a line on which the second binding substance is immobilized, the control line 303 is a line for checking the spreading, and the coloring reagent immobilization line 304 is a line for detecting the reducing agent solution described later. Here, the upstream side and the downstream side mean descriptions intended to indicate the spreading from the upstream side to the downstream side at the time when gold particle composite bodies are spread.

The more specific aspect of the insoluble carrier (or an immunochromatographic kit having the insoluble carrier) include, for example, the insoluble carrier or the immunochromatographic kit disclosed in JP5728453B.

[Insoluble carrier]

The insoluble carrier is preferably a porous carrier. In particular, due to the reason that the effect and the like of the present invention are more excellent, it is preferably a nitrocellulose film (a nitrocellulose membrane), a cellulose membrane, an acetyl cellulose membrane, a polysulfone membrane, a polyether sulfone membrane, a nylon membrane, a glass fiber, a non-woven fabric, a cloth, a thread, or the like is preferable, and a nitrocellulose film is more preferable.

(Second binding substance)

The second binding substance is not particularly limited as long as it is capable of binding to the above antigen.

Specific examples and the suitable aspect of the second binding substance are respectively the same as those of the above-described first binding substance.

The second binding substance may be the same as or different from the above-described first binding substance; however, an aspect in which the second binding substance is a different substance is preferable due to the reason that the effect and the like of the present invention are more excellent.

In addition, in a case where the first binding substance and the second binding substance are antibodies, an aspect in which the antibody which is the first binding substance and the antibody which is the second binding substance are different from each other is preferable due to the reason that the effect and the like of the present invention are more excellent.

Further, in a case where the first binding substance and the second binding substance are antibodies, an aspect in which an epitope (a part of the antigen recognized by the first binding substance) of the first binding substance and an epitope (a part of the antigen recognized by the second binding substance) of the second binding substance are different from each other is preferable due to the reason that the effect and the like of the present invention are more excellent. The difference in epitope between antibodies can be confirmed by, for example, an enzyme-linked immuno-sorbent assay (ELISA).

<Spreading>

The method of spreading gold particle composite bodies on an insoluble carrier having a test line in a state where the gold particle composite bodies are formed is not particularly limited; however, examples thereof include a method in which the above nitrocellulose membrane 300 (or an immunochromatographic kit having the nitrocellulose membrane 300) as illustrated in Fig. 4 is prepared, and the antigen-concentrated solution obtained in the above-described concentration step is dropwise added onto a gold colloid holding pad and moved from the upstream side to the downstream side by using capillary action as illustrated in Fig. 4.

[Capturing step]

The capturing step is a step of capturing the gold particle composite bodies at the reaction site of the insoluble carrier.

As described above, since the second binding substance capable of binding to an antigen is immobilized at the reaction site of the insoluble carrier, the gold particle composite bodies (the composite bodies of an antigen and modified gold particles) spread on the insoluble carrier in the spreading step is captured at the reaction site (the test line) of the insoluble carrier.

The captured gold particle composite bodies are visible since it is colored by the surface plasmon or the like of a gold particle. In addition, it is also possible to estimate the concentration of the captured composite body using an image analysis device or the like. In this way, the antigen in the specimen can be detected.

In a case where a specimen does not contain an antigen, the gold particle composite bodies are not formed, and thus it is not captured at the reaction site of the insoluble carrier and coloration does not occur.

[Silver amplification step]

The silver amplification step is a step of silver-amplifying the gold particle composite body captured in the capturing step.

The silver amplification step is a step of forming large silver particles in the gold particle composite body captured at the reaction site of the insoluble carrier by providing silver ions to the insoluble carrier after the capturing step. More specifically, it is a step in which silver ions are reduced using gold particles of the gold particle composite body as a catalyst to form silver particles (for example, a diameter of 10 $\mu$m or more).

This significantly improves the detection sensitivity of the captured gold particle composite body.

It is noted that the silver amplification step may be carried out together with the spreading step or the silver amplification step may also serve as the spreading step.

<Suitable aspect>

The method of providing silver ions to the insoluble carrier after the capturing step is not particularly limited; however, it is preferably a method in which the following reducing agent solution and the following silver amplification solution are used, due to the reason that the effects and the like of the present invention are more excellent.

Further, in addition to the reducing agent solution and the silver amplification solution, a washing solution may be used to wash the composite body remaining on the insoluble carrier except for the specific binding reaction. The reducing solution may also serve as a washing solution.

(Reducing agent solution)

The reducing agent solution contains a reducing agent capable of reducing silver ions. As the reducing agent capable of reducing silver ions, any inorganic or organic material or a mixture thereof can be used as long as it can reduce silver ions to silver. Preferred examples of the inorganic reducing agent include a reducing metal salt and a reducing metal complex salt, of which the atomic valence is capable of being changed with a metal ion such as $Fe^{2+}$, $V^{2+}$, or $Ti^{3+}$. In a case where an inorganic reducing agent is used, it is necessary to remove or detoxify oxidized ions by complexing or reducing the oxidized ions. For example, in a system in which $Fe^{2+}$ is used as the reducing agent, a complex of $Fe^{3+}$, which is an oxide, is formed using citric acid or ethylenediaminetetraacetic acid (EDTA), and therefore detoxification is possible. In the present invention, it is preferable to use such an inorganic reducing agent, and as a more preferable aspect of the present invention, it is preferable to use a metal salt of $Fe^{2+}$ as the reducing agent.

It is also possible to use, as the reducing agent, a main developing agent (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), or leuco dyes) that is used in a wet-type light-sensitive silver halide photographic material, and other materials obvious to those who are skilled in the technology in the present field, such as a material disclosed in US6020117A.

As the reducing agent, an ascorbic acid reducing agent is also preferable. The useful ascorbic acid reducing agent includes ascorbic acid, an analog thereof, an isomer thereof, and a derivative thereof. Preferred examples thereof include D- or L-ascorbic acid and a sugar derivative thereof (for example, $\gamma$-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly preferred examples thereof include D-, L-, or D,L-ascorbic acid (and an alkali metal salt thereof) or isoascorbic acid (or an alkali metal salt thereof), and a sodium salt is a preferred salt. A mixture of these reducing agents can be used as necessary.

Due to the reason that the effect and the like of the present invention are more excellent, the reducing agent solution is preferably allowed to flow so that the angle between the spreading direction in the spreading step and the spreading direction of the reducing agent solution is 0 degrees to 150 degrees, and more preferably allowed to flow so that the angle between the spreading direction in the spreading step and the spreading direction of the reducing agent solution is 0 degrees to 135 degrees.

Examples of the method of regulating the angle between the spreading direction in the spreading step and the spreading direction of the reducing agent solution include the method described in Examples of JP2009-150869A.

(Silver amplification solution)

The silver amplification solution is a solution containing a compound containing silver ions. As the compound containing silver ions, it is possible to use, for example, organic silver salts, inorganic silver salts, or silver complexes. Preferred examples thereof include silver ion-containing compounds having a high solubility in a solvent such as water, such as silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

As the silver, the organic silver salt, the inorganic silver salt, or the silver complex is preferably contained in the silver amplification solution at a concentration of 0.001 mol/L to 5 mol/L, preferably 0.005 mol/L to 3 mol/L, and more preferably 0.01 mol/L to 1 mol/L.

Examples of the auxiliary agent of the silver amplification solution include a buffer, a preservative such as an antioxidant or an organic stabilizer, and a rate regulating agent. As the buffer, it is possible to use, for example, a buffer formed of acetic acid, citric acid, sodium hydroxide, or one of salts of these compounds, or formed of tris(hydroxymethyl) aminomethane, or other buffers that are used in general chemical experiments. These buffers are appropriately used to adjust the pH of the amplification solution to an optimum pH thereof. In addition, as the antifogging agent, an alkyl amine can be used as an auxiliary agent, and dodecyl amine is particularly preferable. In addition, a surfactant can be used for the intended purpose of improving the solubility of this auxiliary agent, and $C_9H_{19}$-$C_6H_4$-O-$(CH_2CH_2O)_{50}$H is particularly preferable.

Due to the reason that the effect and the like of the present invention are more excellent, the silver amplification solution is preferably allowed to flow from the direction opposite to that of the spreading step described above and more preferably allowed to flow so that the angle between the spreading direction in the spreading step and the spreading direction of the reducing agent solution is 45 degrees to 180 degrees.

Examples of the method of regulating the angle between the spreading direction in the spreading step and the spreading direction of the silver amplification solution include the method described in Examples of JP2009-150869A.

[4] Examination kit

The examination kit according to the embodiment of the present invention is

an examination kit for detecting a high-molecular-weight molecule contained in a biological fluid in a sample solution which is an aqueous solution containing the high-molecular-weight molecule contained in the biological fluid, the examination kit including;
the concentration device according to the embodiment of the present invention described above, and
a detection device that includes an examination strip having an examination region for detecting the high-molecular-weight molecule contained in the biological fluid, a first pot and a second pot in which a first amplification solution and a second amplification solution for amplifying an examination signal in the examination region are enclosed respectively, and a housing case encompassing the examination strip, the first pot, and the second pot

[Concentration device]

The concentration device according to the embodiment of the present invention is as described above.

[Detection device]

The detection device is
a detection device that includes an examination strip having an examination region for detecting the high-molecular-weight molecule contained in the biological fluid, a first pot and a second pot in which a first amplification solution and a second amplification solution for amplifying an examination signal in the examination region are enclosed respectively, and a housing case encompassing the examination strip, the first pot, and the second pot.

[Suitable aspect]

Hereinafter, a suitable aspect of the detection device will be described.

Due to the reason that the effect and the like of the present invention are more excellent, the detection device is preferably an immunochromatographic kit for detecting a test substance (a high-molecular-weight molecule contained in a biological fluid) in a specimen solution (a sample solution), which is an immunochromatographic kit (hereinafter, also referred to as "the immunochromatographic kit according to the embodiment of the present invention" or simply "the immunochromatographic kit") including;

an examination strip including an insoluble carrier having an examination region of a test substance on which a specimen solution is spread,
a first pot and a second pot each having one surface including a sheet member, in which a first amplification solution and a second amplification solution for amplifying a detection signal in the examination region are enclosed respectively, and
a housing case encompassing the examination strip, the first pot, and the second pot,
where the housing case is formed by including a lower case including an accommodating part in which the examination strip is disposed, an upper case joined to the lower case at a peripheral edge, and an intermediate member disposed between the upper case and the lower case,
the intermediate member includes a breaking part that breaks the sheet member of the first pot, with the breaking part facing the sheet member of the first pot, and
the upper case is formed by including a first convex deformation part that is deformed toward the first pot side by applying a pressing force to the portion facing the first pot from the outside and breaks the sheet member of the first pot by the breaking part of the intermediate member, and a second convex deformation part that is deformed toward the second pot side by applying a pressing force to the portion facing the second pot from the outside and breaks the sheet member of the second pot.

In the immunochromatographic kit according to the embodiment of the present invention, it is preferable that by applying a pressing force, the first convex deformation part moves the first pot to a position where the sheet member is broken by the breaking part of the intermediate member.

At this time, it is preferable that the upper case includes two protruding parts erected toward the first pot side, which abut on and moves the first pot in a case where a pressing force is applied to the first convex deformation part.

In the immunochromatographic kit according to the embodiment of the present invention, it is preferable that the first convex deformation part has a centrally symmetric chevron shape.

In addition, at this time, it is preferable that the two protruding parts are disposed symmetrically with respect to the top of the chevron shape.

Further, it is also preferable that the two protruding parts are formed independently with each other on the slope which sandwiches the chevron-shaped top.

In the immunochromatographic kit according to the embodiment of the present invention, in a case where the first convex deformation part includes the above-described two protruding parts, it is preferable that the two protruding parts are disposed symmetrical with respect to the center of the contact surface of the first pot.

In addition, it is preferable that each of the two protruding parts is disposed on the end part side from a position of half of the distance from the center to the end part of the contact surface of the first pot.

It is noted that in the present specification, the convex deformation part means that the convex deformation part is convex-shaped in a case of being viewed from the outside of the immunochromatographic kit, and that similarly, the chevron shape is chevron-shaped in a case of being viewed from the outside.

In the immunochromatographic kit according to the embodiment of the present invention, in a case of including the above-described two protruding parts, the first convex deformation part can be configured to move the first pot while the tip of each of the two protruding parts abuts on the first pot and is gradually displaced toward the end part side.

In the immunochromatographic kit according to the embodiment of the present invention, it is preferable that the bending elastic modulus of the material constituting the first convex deformation part is 50 MPa to 350 MPa.

In addition, it is preferable that the bending elastic modulus of the material constituting the upper case is 50 MPa to 350 MPa and the bending elastic modulus of the material constituting the lower case is 500 MPa to 900 MPa.

In the immunochromatographic kit according to the embodiment of the present invention, it is preferable that the upper case is formed by integrally forming the first convex deformation part and the second convex deformation part by injection molding.

In the immunochromatographic kit according to the embodiment of the present invention, the upper case is formed by including a first convex deformation part that is deformed toward the first pot side by applying a pressing force to the portion facing the first pot from the outside and breaks the sheet member of the first pot by the breaking part of the intermediate member, and a second convex deformation part that is deformed toward the second pot side by applying a pressing force to the portion facing the second pot from the outside and breaks the sheet member of the second pot, and in a case where a person applies a pressing force with a finger or the like to the two convex deformation parts to deform them, it is possible to break the sheet member of the pot, and it is possible to supply the amplification solution to the examination strip, and thus it is possible to normally carry out the amplification reaction without a dedicated analysis apparatus that requires a power source. Accordingly, the immunochromatographic kit according to the embodiment of the present invention is particularly useful in a case where a dedicated analysis apparatus is not provided or in a case of an emergency, a disaster, or the like in which an analysis apparatus cannot be used.

Hereinafter, the embodiment of the immunochromatographic kit according to the embodiment of the present invention will be described with reference to the drawings. However, the immunochromatographic kit according to the embodiment of the present invention is not limited thereto. It is noted that in order to facilitate visual recognition, the scale and the like of each of the components in the drawings are appropriately changed from those of the actual ones.

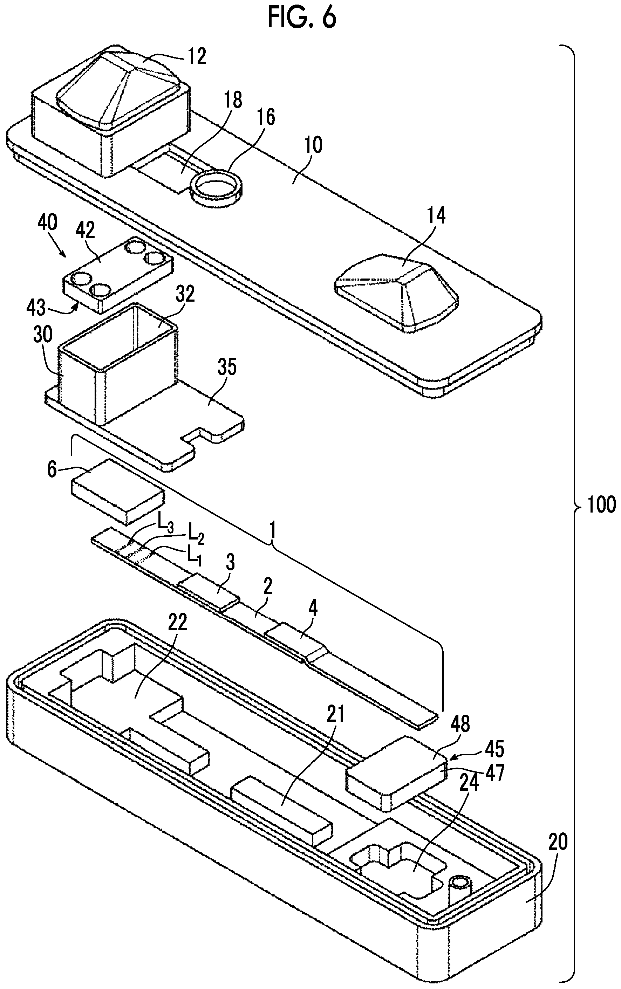

Fig. 5 is a schematic perspective view of an immunochromatographic kit 100 according to the embodiment of the present invention, and Fig. 6 is an exploded schematic perspective view of the immunochromatographic kit 100 of Fig. 5.

As illustrated in Fig. 5 and Fig. 6, the immunochromatographic kit 100 according to the embodiment of the present invention is formed such that a housing case 9 encompasses an examination strip 1 including an insoluble carrier 2 having an examination region of a test substance on which a specimen solution is spread, and a first pot 40 and a second pot 45 each having one surface including a sheet member, in which a first amplification solution 41 and a second amplification solution 46 for amplifying a detection signal in the examination region are enclosed respectively. The housing case 9 is formed by including a lower case 20 including an accommodating part 21 in which the examination strip 1 is disposed, an upper case 10 joined to the lower case 20 at a peripheral edge, and an intermediate member 30 disposed between the upper case 10 and the lower case 20. It is noted that in the description of this immunochromatographic kit 100, the upper case 10 side is defined as the upper side, and the lower case 20 side is defined as the lower side.

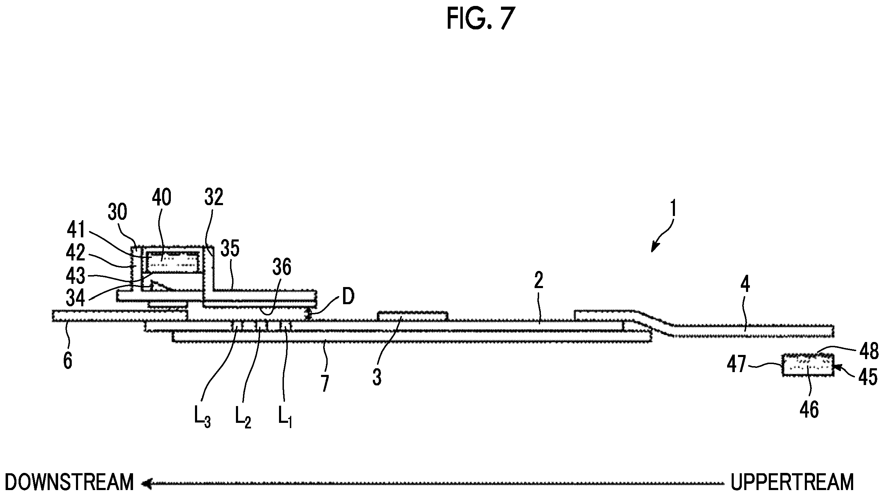

The intermediate member 30 has a pot accommodating part 32 that accommodates the first pot 40 and includes, on the bottom surface, amplification solution filling holes for dropwise adding the first amplification solution 41 onto the insoluble carrier 2. In addition, a protrusion-shaped breaking part 34 that breaks a sheet member 43 is provided at a position in the first pot 40, facing the sheet member 43 in the pot accommodating part 32. In this example, the first pot 40 is disposed above the pot accommodating part 32 so that the surface of the first pot 40, on which the sheet member 43 is provided, is the lower surface, and the breaking part 34 is provided on the bottom surface of the pot accommodating part 32 facing the sheet member 43 (see Fig. 7).

In addition, a flow channel forming part 35 extending toward the downstream side of the bottom surface of the pot accommodating part 32 of the intermediate member 30 is provided. The flow channel forming part 35 is disposed to coincide with an upper position of an examination region $L_1$, a checking region $L_2$, and an amplification indicator region $L_3$, and it is formed of a transparent material in order to make these regions $L_1$ to $L_3$ visible.

The upper case 10 includes a first convex deformation part 12 that is deformed toward the first pot 40 side by applying a pressing force to the portion facing the first pot 40 from the outside and breaks the sheet member 43 of the first pot 40 by the breaking part 34 of the intermediate member 30. In addition, the upper case 10 includes a second convex deformation part 14 that is deformed toward the second pot 45 side by applying a pressing force to the portion facing a second pot 45 from the outside and breaks the sheet member 48 of the second pot 45.

In addition, the upper case 10 includes an opening pore 16 for dropwise addition of a specimen solution, and the specimen solution is dropwise added from the opening pore 16 onto a label holding pad 3 of the examination strip 1. In a case of adjusting the position of the label holding pad 3 so that the positions of the opening pore 16 and the label holding pad 3 match with each other, a specimen solution can be reliably spotted on the label holding pad 3. In addition, the upper case 10 includes an observation window 18 for visually recognizing the three regions $L_1$ to $L_3$, at a position corresponding to the flow channel forming part 35 of the intermediate member 30.

The lower case 20 includes, as an accommodating part in which the examination strip 1 is disposed, an insoluble carrier accommodating part 21 on which the insoluble carrier 2 is placed and an absorption pad accommodating part 22 on which an absorption pad 6 is placed downstream side of the insoluble carrier accommodating part 21. In addition, a second pot accommodating part 24 in which the second pot 45 is accommodated is provided on the upstream side of the insoluble carrier accommodating part 21.

Fig. 7 is a schematic cross-sectional view illustrating a positional relationship between the examination strip 1, the intermediate member 30, and the two pots 40 and 45. As illustrated in Fig. 7, the examination strip 1 includes the insoluble carrier 2 on which a specimen solution is spread, the label holding pad 3 that contains a labeling substance modified with a first substance that is capable of binding to a test substance immobilized on the insoluble carrier 2, a liquid feeding pad 4 that feeds the second amplification solution 46 disposed to be in contact with one end of the insoluble carrier 2 to the insoluble carrier 2, and the absorption pad 6 that is disposed to be in contact with the other end of the insoluble carrier 2. The insoluble carrier 2 is fixedly supported on a back pressure-sensitive adhesive sheet 7. In addition, the insoluble carrier 2 has, between the label holding pad 3 and the absorption pad 6, the examination region $L_1$ containing a second substance that binds to a test substance, the checking region $L_2$ containing a substance that is capable of binding to the first substance, and the amplification indicator region $L_3$ containing a substance that reacts with the second amplification solution, in the order from the label holding pad 3 side.

**EP 4 212 873 B1**

It is noted that in the present specification, the insoluble carrier 2 in which the examination region $L_1$, the checking region $L_2$, and the amplification indicator region $L_3$ are formed may be referred to as a chromatographic carrier. In addition, in the present specification, as described in Fig. 7, the liquid feeding pad 4 side is defined as the upstream side, and the absorption pad 6 side is defined as the downstream side.

The intermediate member 30 is positioned at an upper part on the downstream end side of the examination strip 1, and the first pot 40 is disposed in the pot accommodating part 32 of the intermediate member 30 with the sheet member 43 facing down. The second pot 45 is accommodated below the upstream end of the examination strip 1 of the lower case 20 with the sheet member 48 facing up.

As illustrated in Fig. 7, a gap (a clearance) D is formed between a back surface 36 of the flow channel forming part 35 of the intermediate member 30 and the insoluble carrier 2 of the examination strip 1. The gap D is preferably in a range of 0.01 mm to 1 mm. In a case where it is 0.01 mm or more, the amplification solution or the like can be sufficiently infiltrated, and in a case where it is 1 mm or less, the capillary force is exhibited, whereby the gap between the insoluble carrier 2 and the intermediate member 30 is uniformly filled with the first amplification solution 41.

In the first pot 40 in which the first amplification solution 41 is enclosed, a container 42 having an opening on one surface composed of, for example, a resin material is filled with the first amplification solution 41, and the opening of the container 42 is covered and enclosed by the breakable sheet member 43.

Similarly, in the second pot 45 in which the second amplification solution 46 is enclosed, a container 47 having an opening on one surface composed of, for example, a resin material is filled with the second amplification solution 46, and the opening of the container 47 is covered and enclosed by the breakable sheet member 48.

As the breakable sheet members 43 and 48 in the first pot 40 and the second pot 45, a laminated film such as an aluminum foil or an aluminum laminate sheet is suitably used. Here, "break" refers to a state where a member is not regenerated after being ruptured.

The convex deformation parts 12 and 14 at two places in the upper case will be described in detail.

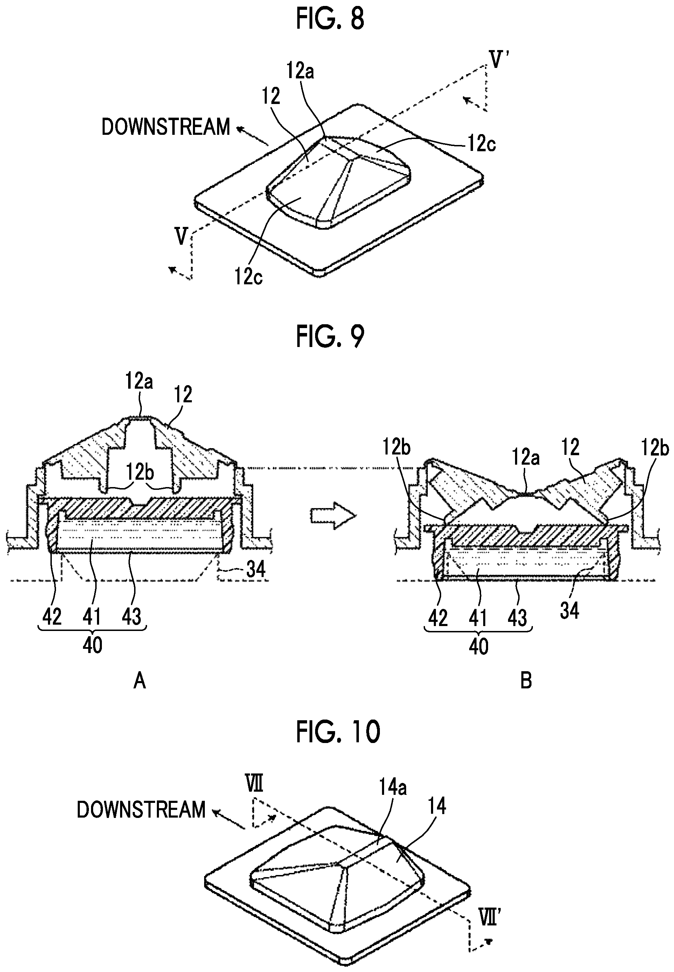

Fig. 8 is a perspective view illustrating the first convex deformation part 12, and Fig. 9 is end views cut along a V-V' line of Fig. 8, where (A) of Fig. 9 illustrates the first convex deformation part 12 before the deformation, and (B) of Fig. 9 illustrates the first convex deformation part 12 after deformation, which are views illustrating a positional relationship with the first pot 40.

In a case of applying a pressing force, the first convex deformation part 12 moves the first pot 40 to a position where the sheet member 43 is broken by the breaking part 34 of the intermediate member 30. Specifically, the first convex deformation part 12 is configured to be pushed downward in a case of being depressed with a finger or the like, and it deforms the first convex deformation part 12 to be downwardly convex (to be recessed part-shaped in a case of being viewed from the outside), thereby moving the first pot 40 toward the breaking part 34 up to a position where the sheet member 43 of the first pot 40 is broken by the breaking part 34 in the pot accommodating part 32 of the intermediate member 30. As a result, the breaking part 34 can break through the sheet member 43 of the first pot 40 and can supply the first amplification solution 41 to the outside. The first amplification solution 41 is dropwise added onto the upper part of the insoluble carrier 2 from the amplification solution filling holes provided on the bottom surface of the pot accommodating part 32 of the intermediate member 30, whereby the first amplification solution 41 can be supplied to the examination region $L_1$, the checking region $L_2$, and the amplification indicator region $L_3$ on the insoluble carrier 2. It is noted that at this time, the gap between the intermediate member 30 and the insoluble carrier 2 is filled with the first amplification solution 41 dropwise added onto the upper part of the insoluble carrier 2 from the amplification solution filling holes, which subsequently passes through the gap and supplied above the examination region $L_1$, the checking region $L_2$, and the amplification indicator region $L_3$, and gradually permeate into the insoluble carrier 2.

As illustrated in Fig. 9, the first convex deformation part 12 includes two protruding parts 12b erected toward the first pot 40 at a position facing the first pot 40. In a case where a pressing force is applied to the first convex deformation part 12 to be deformed, the two protruding parts 12b is configured to abut on the first pot 40 to move the first pot 40.

The first convex deformation part 12 has a centrally symmetric chevron shape, and the two protruding parts 12b are disposed symmetrically with respect to a top 12a of the chevron shape and are formed independently with each other below (on the back surface) the slope 12c which sandwiches the top 12a.

In addition, as illustrated in (A) of Fig. 9, in the first convex deformation part 12 is formed, before the deformation, in the upper case 10 so that the two protruding parts 12b are positioned symmetrically with respect to the center of the contact surface of the first pot 40. In addition, the breaking part 34 of the intermediate member 30 is positioned below the sheet member 43 of the first pot 40, as indicated by a broken line in Fig. 9. In a case where a pressing force is applied to the first convex deformation part 12 to be deformed, the two protruding parts 12b moves the first pot 40 while the tip of each of the two protruding parts 12b abuts on the first pot 40 and is gradually displaced toward the end part side. Then, as illustrated in (B) of Fig. 9, the spacing between the two protruding parts 12b is widened after the first convex deformation part 12 is deformed, and the tips of the two protruding parts 12b are mutually to be positioned on the end part side from a position of half of the distance from the center to the end part of the contact surface of the first pot 40. In the present embodiment, the two protruding parts 12b are independently provided, a gap is provided between the protruding parts 12b (on the back

18

surface of the top 12a), and the first convex deformation part 12 is formed of a flexible material, whereby the first pot 40 is depressed while greatly expanding the gap between the two protruding parts 12b.

The shape and disposition of the protruding parts 12b are not limited to the above-described forms, and the two protruding parts 12b may be provided, for example, before the deformation, at a position which is on the end part side from a position of half of the distance from the center to the end part of the contact surface of the first pot 40.

The first convex deformation part 12 that moves the first pot 40 can move the first pot 40 in parallel since it can evenly push the first pot 40 at two places in a case where the two protruding parts 12b are provided.

The first convex deformation part 12 is easily deformed in a case of being pressed with a finger or the like, and the first convex deformation part 12 becomes downwardly convex (recessed part-shaped). A configuration in which the recessed part shape does not return to the original shape after this pressing and the state where the first pot 40 is pressed can be maintained is preferable. Although the first convex deformation part 12 is configured to press the top 12a, the deformation is also similarly possible due to the elasticity of the first convex deformation part 12 by pressing the chevron-shaped slope.

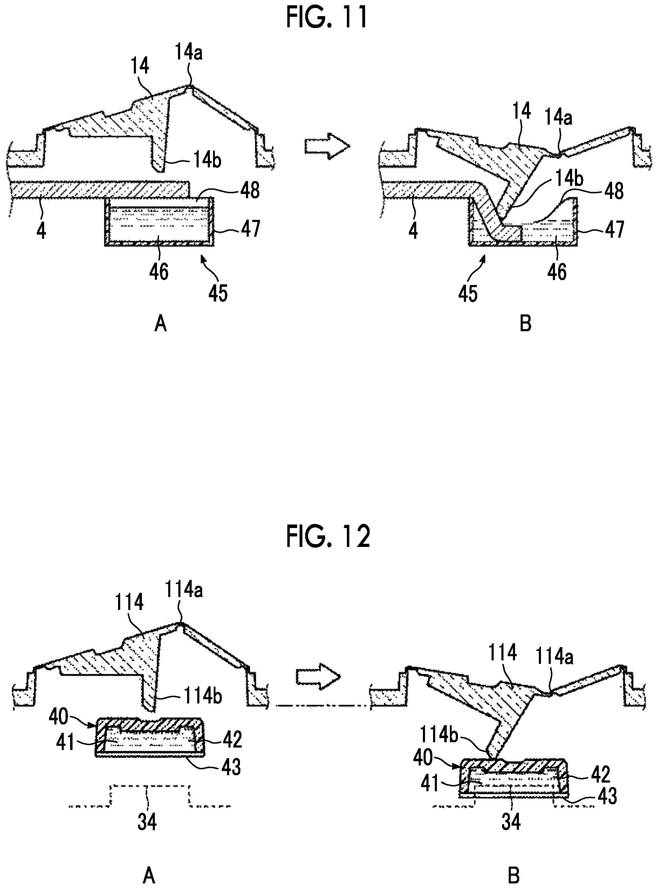

Fig. 10 is a perspective view illustrating the second convex deformation part 14, and Fig. 11 is end views cut along a VII-VII' line of Fig. 10, where (A) of Fig. 11 illustrates the second convex deformation part 14 before the deformation, and (B) of Fig. 11 illustrates the second convex deformation part 14 after deformation, and which are views illustrating together a positional relationship with the second pot 45.

The second convex deformation part 14 breaks the sheet member 48 of the second pot 45 by applying a pressing force. As illustrated in (A) of Fig. 11, the second convex deformation part 14 includes one protruding part 14b erected toward the second pot 45 at a position facing the second pot 45. In addition, the liquid feeding pad 4 of the examination strip 1 is disposed between the second convex deformation part 14 and the second pot 45. A pressing force is applied to the second convex deformation part 14, whereby the second convex deformation part 14 is deformed into a convex shape on the second pot 45 side, that is, into a recessed part shape in a case of being viewed from the outside, and as illustrated in (B) of Fig. 11, the protruding part 14b abuts on the surface of the liquid feeding pad 4 to break through the sheet member 48 of the second pot 45, thereby pushing the liquid feeding pad 4 into the second pot 45. As illustrated in Fig. 11, the second convex deformation part 14 has a chevron shape having a top 14a on a slightly upstream side in the cross section along the direction from the upstream side to the downstream side, and it is configured such that at the time of deformation, the protruding part 14b tilts toward the downstream side to break through the sheet member 48.

By this operation, the liquid feeding pad 4 is immersed in the amplification solution 46 in the second pot 45, and the second amplification solution 46 can permeate into the inside of the liquid feeding pad 4 by capillary action, thereby capable of being supplied to the insoluble carrier 2.

The second convex deformation part 14 is also easily deformed to be recessed part-shaped in a case of being pressed with a finger or the like. A configuration in which the recessed part shape does not return to the original shape after this pressing and the state where the liquid feeding pad 4 is pushed into the second pot 45 can be maintained is preferable.

The present invention realizes a highly sensitive analysis by deforming the first and second convex deformation parts to supply an amplification solution without using a device connected to a power source, where an aspect in which a person carries out the deformation by hand is assumed as one aspect. Accordingly, it is preferable to be designed so that the amplification solution does not accidentally leak to the outside, and it is preferable that the first and second convex deformation parts 12 and 14 which are provided in the upper case 10 are integrally formed without having gaps with other portions of the upper case 10. It is preferable that the convex deformation parts 12 and 14 are made of a stretchable material and are joined to other portions of the upper case 10 in a sealed state. The first and second convex deformation parts 12 and 14 of the upper case 10 and the other portions may be separately produced and then joined to each other. However, it is preferable that the first and second convex deformation parts 12 and 14 are integrally formed by injection molding, as a part of the upper case 10, as one continuous member having no joining portion in the middle.

The first and second convex deformation parts 12 and 14 need to have flexibility such that they can be easily deformed with a human finger or the like. The bending elastic modulus of the material constituting the convex deformation parts 12 and 14 is preferably 50 MPa or more and 350 MPa or less, and more preferably 70 MPa or more and 150 MPa or less.

In addition, in a case where the upper case 10 and the lower case 20 are simply fitted to each other in a case where they are combined, a liquid may leak from the gap, and thus it is preferable that the fitting portion between the upper case 10 and the lower case 20 is also adhered in a sealed state.

As a method of carrying out adhesion between the upper case 10 and the lower case 20, an ultrasonic welding method is preferably used. In general, it is known that welding is difficult to be carried out by ultrasonic welding unless the welding members are made of the same material, and the following combination of the upper case and the lower case is good; polyethylene/polyethylene, polypropylene/polypropylene, or ABS (an acrylonitrile-butadiene-styrene copolymer)/ABS.

On the other hand, in a case where the convex deformation parts 12 and 14 are integrally molded to the upper case 10, it is required that the material constituting the upper case 10 has flexibility. On the other hand, the lower case 20 is preferably rigid in order to fix the examination strip 1 or the second pot 45. Specifically, the bending elastic modulus of the material constituting the upper case 10 is preferably 50 MPa or more and 350 MPa or less, and more preferably 70 MPa or more and 150 MPa or less. The bending elastic modulus of the material constituting the lower case 20 is preferably 500 MPa or more and 900 MPa, and particularly preferably 650 MPa or more and 750 MPa or less.

It is noted that the bending elastic modulus is a value calculated according to Expression (1) as follows in an environment of a temperature of 20°C according to the measuring method of ISO178 standard.

A plate-shaped test piece having a width b (mm) and a thickness h (mm) is prepared for a material for which the bending elastic modulus is to be measured, and the test piece is supported by two fulcrums where the distance between the fulcrums is L (mm). A load of F (N) is applied to the center between the fulcrums, and the amount of deflection (mm) in the direction in which the load is applied is measured. A deflection-load curve is created, where the horizontal axis indicates the deflection S (mm) and the vertical axis indicates the load F (N). A tangent line at the origin of this curve is determined, and a slope thereof ($\Delta F/\Delta S$) in a case where the amount of change in load is denoted by $\Delta F$ (N) and the amount of change in deflection is denoted by $\Delta S$ (mm)) is calculated whereby the bending elastic modulus E (MPa) can be calculated by using the following expression.

$$\text{Bending elastic modulus E} = (L^3/(4bh^3)) \times (\Delta F/\Delta S) \cdots \text{Expression (1)}$$

Accordingly, the combination of the upper case and the lower case is most preferably a combination of polypropylene containing a softening agent and polypropylene. Here, the softening agent that is used in the polypropylene containing a softening agent is preferably an olefin-based elastomer, where the concentration of the olefin-based elastomer with respect to polypropylene is preferably 20% by mass or more and 60% by mass or less, and particularly preferably 40% by mass or more and 55% by mass or less. Specific examples of the softening agent include TAFTHREN (registered trade name) manufactured by Sumitomo Chemical Co., Ltd.

It is noted that the immunochromatographic kit according to the embodiment of the present invention only needs to have two or more convex deformation parts, and in a case where there are three or more kinds of solutions to be supplied to the examination strip, three or more convex deformation parts may be correspondingly provided.

As the insoluble carrier 2, it is possible to use, for example, a nitrocellulose membrane. In addition, the back pressure-sensitive adhesive sheet 7 on which the insoluble carrier 2 is fixed is a sheet-shaped base material on which a surface to which the insoluble carrier 2 is attached is a pressure-sensitive adhesive surface.

The label holding pad 3 is fixed at a central portion of the insoluble carrier 2 in the longitudinal direction. As the labeling substance, it is possible to use, for example, a gold colloid having a diameter of 50 nm (EM. GC50, manufactured by BBI Solutions Inc.). In a case of modifying the surface of the labeling substance with a substance that binds to a test substance, it is possible to form a conjugate with the test substance.

The labeling substance is not limited to the above, and a metal sulfide that can be used in a general chromatograph method, coloring particles that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, and a silver colloid is preferable since it exhibits a yellow color, among which a gold colloid is most preferable.

It is noted that the examination strip 1 is positioned such that the position of the opening pore 16 for dropwise addition of a specimen solution of the upper case 10 and the position of the label holding pad 3 match with each other.

The examination region $L_1$ is a labeling substance supplementing region which contains the second substance that binds to a test substance and in which the labeling substance that has bound to the test substance is supplemented through the test substance. For example, in a case where it is desired to detect an influenza A type virus or a biomarker thereof as a test substance, an aspect in which the examination region $L_1$ is constituted by an antibody immobilization line on which, for example, an anti-influenza A type monoclonal antibody (Anti-influenza A SPTN-5 7307, manufactured by Medix Biochemica) has been immobilized in a line shape by physical adsorption is preferable.

In a case where a composite body in which the test substance binds to the labeling substance through the first substance reaches the examination region $L_1$, the second substance specifically binds to the test substance, whereby the labeling substance is supplemented through the test substance and the first substance. On the other hand, the labeling substance that does not constitute the composite body with the test substance passes through the examination region $L_1$ without being supplemented.

The checking region $L_2$ is a region for checking the completion of the spreading of the specimen solution, which contains a substance that is capable of binding to the first substance, the substance being spread in the insoluble carrier 2 together with the specimen solution from the label holding pad 3, and in which the labeling substance that has passed through the examination region $L_1$ is supplemented through the first substance. For example, in a case where it is desired to detect an influenza A type virus or a biomarker thereof as a test substance, an aspect in which for example, an anti-mouse IgG antibody (Anti-mouse IgG (H + L), rabbit F(ab')2, product number: 566-70621, manufactured by FUJIFILM Wako Pure Chemical Corporation) is immobilized in a line shape by physical adsorption is preferable.

The amplification indicator region $L_3$ is a region that serves as an indicator of the timing of the dropwise addition of the first amplification solution 41, which contains a substance that reacts with the second amplification solution 46, the substance reacting with the second amplification solution 46 to develop a color or change a color, thereby indicating that the second amplification solution 46 has been spread to the amplification indicator region $L_3$. For example, in a case where a mixed aqueous solution of an iron nitrate aqueous solution and citric acid (manufactured by Fujifilm Wako Pure Chemical Corporation, 038-06925) is used as the second amplification solution, an aspect in which the amplification indicator region $L_3$ is constituted by a coloring reagent immobilization line on which Bromocresol Green (manufactured by FUJIFILM Wako Pure Chemical Corporation) has been immobilized in a line shape is preferable. At this time, in a case where the second amplification solution 46 reaches the amplification indicator region $L_3$, the region $L_3$ changes from a green color to an orange color. This discoloration can be regarded as an indicator that the examination region $L_1$ and the checking region $L_2$ are sufficiently filled with the second amplification solution 46.

As a method of amplifying a signal of a metal-based labeling substance such as a metal colloid, it is preferable to use a method (hereinafter, silver amplification) in which silver ions and a reducing agent for silver ions are brought into contact with a labeling substance, the silver ions are reduced by the reducing agent to generate silver particles, and the silver particles are deposited on the labeling substance with the labeling substance as a nucleus.

In order to realize the silver amplification, a solution containing silver ions may be used as the first amplification solution 41, and a reducing agent solution containing a reducing agent for silver ions may be used as the second amplification solution 46.

(First amplification solution)

The solution containing silver ions, which is used as the first amplification solution 41, is preferably a solution obtained by dissolving a silver ion-containing compound in a solvent. As the compound containing silver ions, it is possible to use, for example, an organic silver salt, an inorganic silver salt, or a silver complex. It is preferably an inorganic silver salt or a silver complex. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

(Second amplification solution)

As the reducing agent that is used in the reducing agent solution containing a reducing agent capable of reducing silver ions, where the reducing agent solution is used as the second amplification solution 46, any inorganic or organic material or a mixture thereof can be used as long as it can reduce silver ions to silver. Preferred examples of the inorganic reducing agent include a reducing metal salt and a reducing metal complex salt, of which the atomic valence is capable of being changed with a metal ion such as $Fe^{2+}$, $V^{2+}$, or $Ti^{3+}$. In a case where an inorganic reducing agent is used, it is necessary to remove or detoxify oxidized ions by complexing or reducing the oxidized ions. For example, in a system in which $Fe^{2+}$ is used as the reducing agent, a complex of $Fe^{3+}$, which is an oxide, is formed using citric acid or ethylenediaminetetraacetic acid (EDTA), which enables the detoxification. In this system, it is preferable to use such an inorganic reducing agent, where more preferably, a metal salt of $Fe^{2+}$ is preferable.

It is also possible to use a main developing agent used in a light-sensitive silver halide photographic material of a wet-type (such as methyl gallate, hydroquinone, substituted hydroquinone, 3-pyrazolidones, p-aminophenols, p-phenylenediamines, hindered phenols, amidoximes, azines, catechols, pyrogallols, ascorbic acid (or derivatives thereof), and leuco dyes), and other materials obvious to those who are skilled in the related art in the present field, such as a material disclosed in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, $\gamma$-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly preferred examples thereof include D-, L-, or D,L-ascorbic acid (and an alkali metal salt thereof) or isoascorbic acid (or an alkali metal salt thereof), and a sodium salt is a preferred salt. A mixture of these reducing agents can be used as necessary.

It is noted that in the present embodiment, although the first convex deformation part 12 is configured to move the first pot 40 toward the breaking part 34 provided in the intermediate member 30, the first convex deformation part 12 may be configured such that the sheet member 43 of the first pot 40 can be broken by the breaking part 34 in association with the deformation of the first convex deformation part 12.

The configuration of the accommodating part 32 accommodating the first pot 40 and the first pot 40 is not limited to the configuration of the present embodiment either as long as it is a configuration in which the first amplification solution 41 that flows out of the first pot 40 by the sheet member 43 of the first pot 40 being broken can be dropwise added onto the insoluble carrier 2 from the amplification solution filling holes on the bottom surface of the accommodating part 32.

In addition, it is preferable that there are two or more protruding parts of the first convex deformation part, since the first pot 40 can be moved in parallel without being tilted. However, the first convex member may have a form in which one protruding part having the same shape as the second convex deformation part of the above embodiment is provided. The convex deformation part having the same shape as the second convex part deformation part may be used as the first convex deformation part that moves the first pot 40.

Fig. 12 is a cut end view similar to Fig. 11 illustrating a form in which the deformation part 114 having the same shape as the second convex deformation part is used for moving the first pot 40.

As illustrated in (A) of Fig. 12, before the deformation, the first pot 40 is disposed to be positioned below the protruding part 114b of the convex deformation part 114. In addition, the breaking part 34 of the intermediate member 30 is positioned below the first pot 40. In a case of depressing the top 114a of the convex deformation part 114, the protruding part 114b presses against the upper surface of the first pot 40 and pushes down the first pot 40. As a result, the breaking part 34 breaks through the sheet member 43 of the first pot 40, and the first amplification solution 41 enclosed in the first pot 40 flows out of the first pot and is supplied to the examination strip 1.

In this way, the pot can be moved even in a case where there is only one protruding part provided in the convex deformation part 114.

It is noted that the immunochromatographic kit according to the present invention may include a set of equipment or a part thereof necessary for an examination, such as a kit that encompasses a pot containing a sample extraction solution containing an auxiliary agent that assists the extraction of the sample or a pot containing a sample diluent, a desiccant or an oxygen scavenger, which assists the storage of the kit, an attached document such as an instruction manual, and a sample collection instrument such as a cotton swab.

In a case of using this immunochromatographic kit of the present invention, it is possible to carry out an examination with high accuracy by the kit itself without using a dedicated analysis apparatus or the like.

<Immunochromatographic examination method>

An immunochromatographic examination method using the immunochromatographic kit 100 will be briefly described.

A specimen solution is dropwise added onto the label holding pad 3 from the opening pore 16 for dropwise addition of a specimen solution. In a case where the test substance is contained in the specimen solution, a composite body of the test substance and the labeling substance is formed through the first substance by binding the test substance to the first substance in the label holding pad 3, and this composite body is spread together with the specimen solution toward the absorption pad 6 side by capillary action due to the suction force of the absorption pad 6. Simultaneously with or after the dropwise addition of this specimen solution, the second convex deformation part 14 is depressed to displace the liquid feeding pad 4, thereby breaking the sheet member 48 of the second pot 45, and the liquid feeding pad 4 is immersed in the second amplification solution 46 to send the second amplification solution 46 to the insoluble carrier 2. It is noted that the timing of depressing the second convex deformation part 14 is preferably within 30 seconds from the time of the dropwise addition of the specimen solution, and particularly preferably immediately after the dropwise addition of the specimen solution.

The composite body that has reached the examination region $L_1$ binds to the second substance in the examination region $L_1$, thereby being captured. In addition, the first substance that does not bind to the test substance passes through the examination region $L_1$, reaches the checking region $L_2$, and binds to the substance that binds to the first substance in the checking region $L_2$, thereby being captured.

The second amplification solution 46 goes through the examination region $L_1$ and the checking region $L_2$ and reaches the amplification indicator region $L_3$. At this time, the amplification indicator region $L_3$ is discolored, whereby the arrival of the second amplification solution 46 at the amplification indicator region $L_3$ can be visually recognized. After the checking of the discoloration of the amplification indicator region $L_3$, the first convex deformation part 12 is depressed to supply the first amplification solution 41 onto the insoluble carrier 2.

After the first amplification solution 41 is supplied to the insoluble carrier 2, the reaction is waited for completion, and then the color development of the examination region $L_1$ and the checking region $L_2$ is checked through the observation window 18. It is possible to check the presence or absence of the test substance and the highness or lowness of the concentration thereof by the color development of the examination region $L_1$, and it is possible to check whether or not the examination for measuring the test substance is successful, by the color development of the checking region $L_2$. The color development in the examination region $L_1$ and the checking region $L_2$ is obtained by amplifying the signal of the label, and a highly sensitive examination can be realized.

Examples

Hereinafter, the present invention will be described in more detail with reference to Examples; however, the present invention is not limited thereto.

[A] Case where antigen is LAM

[Preparation of sample solution]

Lipoarabinomannan (LAM) (02249-61, Nacalai Tesque, Inc.) (an antigen) extracted from tubercle bacillus was added to a urine sample obtained by pooling urine samples (BioIVT LLC) of healthy subjects to prepare a sample solution (an antigen-containable solution) having a LAM concentration of 0.1 ng/ml.

[Example A1]

[Production of concentration device]

A concentration device of Example A1 was produced as follows.
First, a mini-centrifugal filtration filter (VIVACLEAR MINI 0.8 μm, the material of the porous membrane: polyether sulfone (PES), the hole diameter of the porous membrane: 0.8 μm, manufactured by Sartorius AG) was prepared. As illustrated in Fig. 2, the centrifugal filtration filter includes the first container 210 and the second container 220, and a bottom part of the first container 210 is constituted of the discharge unit 212 consisting of a porous membrane. The surface energy of PES, which is the material of the porous membrane, is $46.10^{-3}$ N/m (46 dynes/cm).
Next, 100 mg of commercially available SAP particles (AQUALIC CA, manufactured by Nippon Shokubai Co., Ltd., swelling ratio: 30 g/g, particle diameter: 300 μm) were placed in the first container of the mini-centrifugal filtration filter.
In this way, such a concentration device as illustrated in Fig. 1 was obtained.

[Concentration of sample solution]

Using the obtained concentration device, the above-described sample solution was concentrated as follows.

<Sample solution injection step>

700 μL of the above-described sample solution (the sample solution having a LAM concentration of 0.1 ng/mL) was injected into the first container of the obtained concentration device and mixed with the SAP particles (Fig. 3A). It is noted that the injected sample solution was held in the first container without being discharged from the discharge unit.

<Water absorption step>

Then, it was allowed to stand for 5 minutes. During this period, the solution (the water or the low-molecular-weight molecule) contained in the sample solution was absorbed by the SAP particles to generate a sample solution concentrated solution, which was a concentrated solution of the sample solution, in the first container (Fig. 3B).

<Discharge step>

Next, the concentration device was placed in a small microcentrifuge (Multi-Spin, manufactured by Tommy Seiko Co., Ltd.), and a centrifugal force (rotation speed: 6,000 rpm), time: several tens of seconds) was applied to the sample solution concentrated solution in the direction from the top to the bottom (the direction of the discharge unit) to discharge the sample solution concentrated solution from the porous membrane (the discharge unit) (Fig. 3C).

<Recovery step>

The discharged sample solution concentrated solution was recovered in a second container (Fig. 3D). The amount of the obtained sample solution concentrated solution was 100 μL. The concentration time (the time (minutes) taken from the sample solution injection step to the recovery step) was 7 minutes.

[Detection of LAM]

The obtained sample solution concentrated solution (60 μL) was subjected to the detection of LAM using a commercially available LAM kit (Alere Determine™ TB LAM Ag, manufactured by Alere Inc.), and then the optical density was measured using LAS4000 manufactured by FUJIFILM Corporation.
In addition, aqueous solutions respectively having a LAM concentration of 0.1 ng/mL, 0.2 ng/mL, 0.3 ng/mL, and 0.4 ng/mL were prepared, and the optical density was measured in the same manner to create a calibration curve. Then, using the calibration curve, the LAM concentration (after concentration) of the above-described sample solution concentrated solution, was determined from the optical density thereof to calculate the LAM concentration rate (= LAM concentration (after concentration)/LAM concentration (before concentration). The LAM concentration rate is shown in Table 1. In a case where the LAM concentration rate is more than 1, it means that the sample solution has been concentrated. It is preferable that the LAM concentration rate is high.

[Example A2]

The porous membrane (material: PES) was taken out from the mini-centrifugal filtration filter used in Example A1, and instead, a porous membrane (material: cellulose acetate (CA), hole diameter: 5 μm) (a CA membrane taken out from Minisart CA 5 μm, manufactured by Advantech Co., Ltd.) was installed. As illustrated in Fig. 2, the mini-centrifugal filtration filter after the porous membrane replacement includes the first container and the second container, and a bottom part of the first container is constituted of the porous membrane (the discharge unit). The surface energy of CA, which is the material of the porous membrane, is $50.10^{-3}$ N/m (50 dynes/cm).
Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.
In this way, such a concentration device as illustrated in Fig. 1 was obtained.
Using the obtained concentration device, the concentration of the sample solution and the detection of the LAM were carried out in the same manner as in Example A1. The LAM concentration rate is shown in Table 1.
The amount of the obtained sample solution concentrated solution was 100 μL, and the concentration time was 7 minutes.

[Example A3]

The porous membrane (material: PES) was taken out from the mini-centrifugal filtration filter used in Example A1, and instead, a porous membrane (material: cellulose acetate (CA), hole diameter: 0.2 μm) (a CA membrane taken out from Minisart CA 0.2 μm, manufactured by Advantech Co., Ltd.) was installed. As illustrated in Fig. 2, the mini-centrifugal filtration filter after the porous membrane replacement includes the first container and the second container, and a bottom part of the first container is constituted of the porous membrane (the discharge unit). The surface energy of CA, which is the material of the porous membrane, is $50.10^{-3}$ N/m (50 dynes/cm).
Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.
In this way, such a concentration device as illustrated in Fig. 1 was obtained.
Using the obtained concentration device, the concentration of the sample solution and the detection of the LAM were carried out in the same manner as in Example A1. The LAM concentration rate is shown in Table 1.
The amount of the obtained sample solution concentrated solution was 100 μL, and the concentration time was 7 minutes.

[Example A4]

The porous membrane (material: PES) was taken out from the mini-centrifugal filtration filter used in Example A1, and instead, a porous membrane (material: glass fiber (GF), hole diameter: 0.45 μm) (a GF membrane taken out from Puradisc 13, manufactured by Cytiva) was installed. As illustrated in Fig. 2, the mini-centrifugal filtration filter after the porous membrane replacement includes the first container and the second container, and a bottom part of the first container is constituted of the porous membrane (the discharge unit). The surface energy of GF, which is the material of the porous membrane, is $73.10^{-3}$ N/m (73 dynes/cm).
Next, 100 mg of commercially available SAP particles (AQUALIC CA, manufactured by Nippon Shokubai Co.,

Ltd., swelling ratio: 30 g/g) after the porous membrane replacement were placed in the first container of the mini-centrifugal filtration filter.

In this way, such a concentration device as illustrated in Fig. 1 was obtained.

Using the obtained concentration device, the concentration of the sample solution and the detection of the LAM were carried out in the same manner as in Example A1. The LAM concentration rate is shown in Table 1.

The amount of the obtained sample solution concentrated solution was 100 $\mu$L, and the concentration time was 7 minutes.

[Example A5]

The porous membrane (material: PES) was taken out from the mini-centrifugal filtration filter used in Example A1, and instead, a porous membrane (material: hydrophilic PTFE, hole diameter: 0.05 $\mu$m) (a PTFE membrane manufactured by Advantech Co., Ltd.) was installed. As illustrated in Fig. 2, the mini-centrifugal filtration filter after the porous membrane replacement includes the first container and the second container, and a bottom part of the first container is constituted of the porous membrane (the discharge unit). The surface energy of hydrophilic PTFE, which is the material of the porous membrane, is $35.10^{-3}$ N/m (35 dynes/cm).

Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.

In this way, such a concentration device as illustrated in Fig. 1 was obtained.

Using the obtained concentration device, the concentration of the sample solution and the detection of the LAM were carried out in the same manner as in Example A1. The LAM concentration rate is shown in Table 1.

The amount of the obtained sample solution concentrated solution was 100 $\mu$L, and the concentration time was 7 minutes.

[Example A6]

The porous membrane (material: PES) was taken out from the mini-centrifugal filtration filter used in Example A1, and instead, a porous membrane (material: hydrophobic PTFE, hole diameter: 0.05 $\mu$m) (a PTFE membrane manufactured by Advantech Co., Ltd.) was installed. As illustrated in Fig. 2, the mini-centrifugal filtration filter after the porous membrane replacement includes the first container and the second container, and a bottom part of the first container is constituted of the porous membrane (the discharge unit). The surface energy of hydrophobic PTFE, which is the material of the porous membrane, is $23.10^{-3}$ N/m (23 dynes/cm).

Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.

In this way, such a concentration device as illustrated in Fig. 1 was obtained.

Using the obtained concentration device, the concentration of the sample solution and the detection of the LAM were carried out in the same manner as in Example A1. The LAM concentration rate is shown in Table 1.

The amount of the obtained sample solution concentrated solution was 60 $\mu$L, and the concentration time was 7 minutes. It is conceived that the reason why the amount of the obtained sample solution concentrated solution is small is that the hole diameter of the porous membrane is small.

[Example A7]

A concentration device was produced according to the same procedure as in Example A1.

The concentration of the sample solution and the detection of the LAM were carried out in the same manner as in Example A1, except that the above-described sample solution was diluted 4-fold with a pooled urine sample to prepare a sample solution (a solution in which an antigen is containable) having a LAM concentration of 0.025 ng/mL and this sample solution was used. The LAM concentration rate is shown in Table 1.

[Comparative Example A1]

The porous membrane (material: PES) was taken out from the mini-centrifugal filtration filter used in Example A1, and instead, a porous membrane (material: glass fiber (GF), hole diameter: 100 $\mu$m) (a GF membrane manufactured by Merck KGaA) was installed. As illustrated in Fig. 2, the mini-centrifugal filtration filter after the porous membrane replacement includes the first container and the second container, and a bottom part of the first container is constituted of the porous membrane (the discharge unit). The surface energy of GF, which is the material of the porous membrane, is 73.10⁻³ N/m (73 dynes/cm).

Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.

In this way, such a concentration device as illustrated in Fig. 1 was obtained.

As a result of making an attempt to carry out the concentration of the sample solution in the same manner as in Example A1, by using the obtained concentration device, the sample solution injected into the first container was entirely discharged from the discharge unit, without being held in the first container, and recovered in the second container. The recovered sample solution was 700 μL. The sample solution recovered in the second container was subjected to the detection of LAM in the same manner as in Example A1. The LAM concentration rate is shown in Table 1.

[Comparative Example A2]

The porous membrane (material: PES) was taken out from the mini-centrifugal filtration filter used in Example A1, and instead, a porous membrane (material: cellulose, hole diameter: less than 0.01 μm) (a membrane taken out from Amicon Ultra 3kDa, 0.5 ml, manufactured by Merck KGaA) was installed. As illustrated in Fig. 2, the mini-centrifugal filtration filter after the porous membrane replacement includes the first container and the second container, and a bottom part of the first container is constituted of the porous membrane (the discharge unit). The surface energy of GF, which is the material of the porous membrane, is 73.10⁻³ N/m (73 dynes/cm).

Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.

In this way, such a concentration device as illustrated in Fig. 1 was obtained.

As a result of making an attempt to carry out the concentration of the sample solution in the same manner as in Example A1, by using the obtained concentration device, the sample solution concentrated solution was not discharged from the porous membrane by centrifugation for a short time in the discharge step, and thus the sample solution concentrated solution could not be recovered. As a result, LAM was not detected.

[Comparative Example A3]

Instead of the concentration device of Example A1, a mini-centrifugal filtration filter (VIVACLEAR MINI 0.8 μm, the material of the porous membrane: polyether sulfone (PES), the hole diameter of the porous membrane: 0.8 μm, manufactured by Sartorius AG) (a filter without having SAP particles) was used to carry out the sample solution injection step, the discharge step, and the recovery step in the same manner as in Example A1. The entire sample solution injected into the first container was recovered in the second container. The amount of the recovered sample solution was 700 μL. The recovered sample solution was subjected to the detection of LAM in the same manner as in Example A1. The LAM concentration rate is shown in Table 1.

[Comparative Example A4]

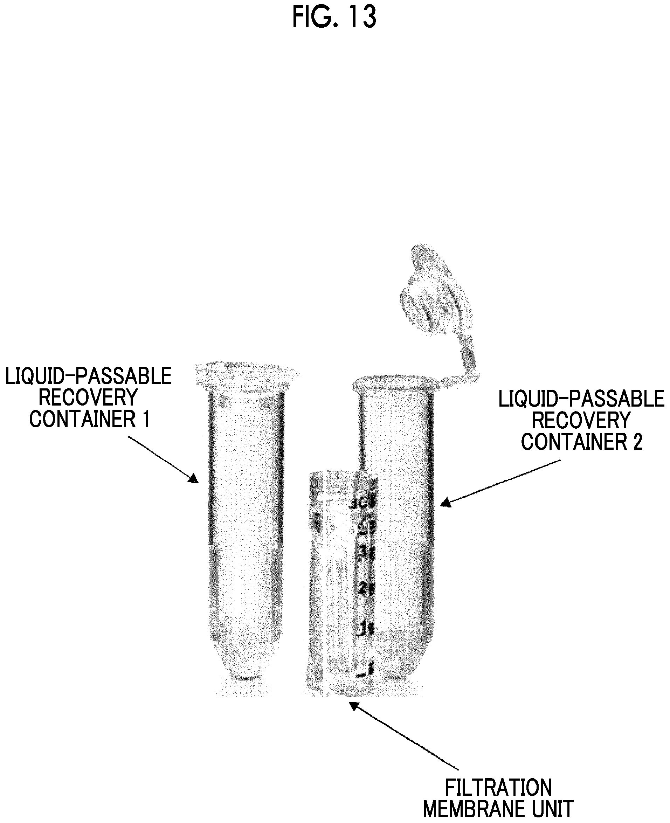

The above-described sample solution was concentrated using Amicon Ultra (3k (3k is 3 KDa and a fractional molecular weight)) (manufactured by Merck KGaA; UFC5003BK). As illustrated in Fig. 13, the product consists of a filtration membrane unit and a liquid-passable recovery container. It is a product that recovers a concentrated solution from the filtration membrane unit by carrying out centrifugation in a state where the filtration membrane unit is inserted into the liquid-passable recovery container (having no filtration membrane). In a method of recovering a concentrated solution remaining in the filtration membrane unit, a filtration membrane unit is inserted into the liquid-passable recovery container with the direction of insertion into the recovery container being opposite to the direction at the time of the first centrifugation, and centrifugation is carried out at a low rotation speed, whereby the concentrated solution can be recovered.

The filtration membrane that is used in the filtration membrane unit is manufactured by Millipore Corporation, where a low-adsorption regenerated cellulose membrane is used. It is a membrane presumed to have a hole diameter of 0.05 μm or less since the fractional molecular weight is 3KDa although the hole diameter thereof is not described. There are two liquid-passable recovery containers, one of which is a container for recovering or discarding a liquid that has passed through the filtration membrane unit. The other is a container for recovering the remaining concentrated solution in the filtration membrane unit.

700 μL of a sample solution having a LAM concentration of 0.1 ng/mL was placed in a filtration membrane unit of such a product. It was subjected to centrifugation at a rotation speed of 8,000 rpm at 4°C for 20 minutes in a state of being inserted into a liquid-passable recovery container 1. At this time, 370 μL of the liquid moved to the liquid-passable recovery container side, and a small amount of the liquid remained in the filtration membrane unit. The liquid-passable recovery container was replaced with a new container (a liquid-passable recovery container 2), and an ultrafiltration unit was inserted into the liquid-passable recovery container with the direction of insertion into the recovery container being opposite to the direction at the time of the first centrifugation and subjected to centrifugation at 2,000 rpm for 2 minutes (referred to as reverse spin centrifugation). As a result, the amount of the obtained sample solution concentrated solution was 130 μL. At this time, the time required for concentration was 26 minutes. The obtained sample solution concentrated solution was subjected to the detection of LAM in the same manner as in Example A1. The LAM concentration rate is shown in Table 1.

[Comparative Example A5]

The porous membrane (material: PES) was taken out from the mini-centrifugal filtration filter used in Example A1, and instead, a porous membrane (material: polypropylene (PP), hole diameter: 50 μm) (a fine polypropylene mesh, manufactured by AS ONE Corporation) was installed. As illustrated in Fig. 2, the mini-centrifugal filtration filter after the porous membrane replacement includes the first container and the second container, and a bottom part of the first container is constituted of the porous membrane (the discharge unit). The surface energy of GF, which is the material of the porous membrane, is $73.10^{-3}$ N/m (73 dynes/cm).
Next, 100 mg of commercially available SAP particles (AQUALIC CA, manufactured by Nippon Shokubai Co., Ltd., swelling ratio: 30 g/g) after the porous membrane replacement were placed in the first container of the mini-centrifugal filtration filter.
In this way, such a concentration device as illustrated in Fig. 1 was obtained.
As a result of making an attempt to carry out the concentration of the sample solution in the same manner as in Example A1, by using the obtained concentration device, the sample solution injected into the first container was discharged from the discharge unit, without being held in the first container, and recovered in the second container. The sample solution recovered in the second container was subjected to the detection of LAM in the same manner as in Example A1. The LAM concentration rate is shown in Table 1.

[Reference Example A6]

A concentration device was produced according to the same procedure as in Comparative Example A3.
The sample solution injection step, the discharge step, and the recovery step were carried out in the same manner as in Comparative Example A3, except that the above-described sample solution was diluted 4-fold with a pooled urine sample to prepare a sample solution (a solution in which an antigen is containable) having a LAM concentration of 0.025 ng/mL and this sample solution was used. The entire sample solution injected into the first container was recovered in the second container. The amount of the recovered sample solution was 700 μL. The recovered sample solution was subjected to the detection of LAM in the same manner as in Example A1; however, the results were equal to or lower than the detection limit, and the concentration could not be checked. Since the LAM was not concentrated, it is described as 1-fold in Table 1.

[Table 1]

| | Example A1 | Example A2 | Example A3 | Example A4 | Example A5 | Example A6 | Example A7 | Comparative Example A1 | Comparative Example A2 | Comparative Example A3 | Comparative Example A4 | Comparative Example A5 | Comparative Example A6 |
|---|---|---|---|---|---|---|---|---|---|---|---|---|---|
| SAP swelling ratio [g/g] | 30 | 30 | 30 | 30 | 30 | 30 | 30 | 30 | 30 | - | - | 30 | - |
| LAM concentration (before concentration) [ng/ml] | 0.1 | 0.1 | 0.1 | 0.1 | 0.1 | 0.1 | 0.025 | 0.1 | 0.1 | 0.1 | 0.1 | 0.1 | 0.025 |
| Porous membrane material | PES | CA | CA | GF | | | PES | GF | CA | PES | CA | PP | PES |
| Pore diameter [μm] | 0.8 | 5 | 0.2 | 0.45 | 0.05 | 0.05 | 0.8 | 100 | <0.01 | 0.8 | | 50 | 0.8 |
| Surface energy $10^{-3}$ N/m [dynes/cm] | 46 | 50 | 50 | 75 | 35 | 23 | 46 | 73 | 50 | 46 | 50 | 50 | 46 |
| Number of times of centrifugation | 1 | 1 | 1 | 1 | 1 | 1 | 1 | - | 1 | 1 | 2 | - | 1 |
| Concentration time [minute] | 7 | 7 | 7 | 7 | 7 | 7 | 7 | - | - | 7 | 26 | - | 7 |
| LAM concentration rate | 4 | 4 | 4 | 4 | 3.8 | 2.5 | 4 | 1 | - | 1 | 4 | 1 | 1 |

In Table 1, the "SAP swelling ratio" indicates the swelling ratio [g/g] of the super absorbent polymer used. The method of measuring the swelling ratio is as described above.

In Table 1, the "porous membrane material" indicates the material of the porous membrane used, PES indicates polyether sulfone, CA indicates cellulose acetate, GF indicates glass fiber, hydrophilic PTFE indicates hydrophilic polytetrafluorocthylene, hydrophobic PTFE indicates hydrophobic polytetrafluoroethylene, and PP indicates polypropylene.

In Table 1, the "hole diameter" indicates the hole diameter [μm] of the porous membrane used.

In Table 1, the "surface energy" indicates the surface energy [N/m (dynes/cm)] of the material of the porous membrane used. The method of measuring the surface energy is as described above.

In Table 1, the "concentration time" indicates the time (minutes) taken from the sample solution injection step to the recovery step. Practically, the concentration time is preferably 20 minutes or less.

As can be seen from Table 1, in a case where Examples A1 to A7, which are the concentration devices according to the embodiment of the present invention, were used, the sample solution could be concentrated in a short time (20 minutes or less). Among them, Examples A1 to A5 and Examples A7, in which the porous membrane consisted of a material having a surface energy of $24.10^{-3}$ N/m (24 dynes/cm) or more and $75.10^{-3}$ N/m (75 dynes/cm) or less, exhibited a higher concentration rate. Among them, Examples A1 to A4, in which the porous membrane consisted of a material having a surface energy of $40.10^{-3}$ N/m (40 dynes/cm) or more and $75.10^{-3}$ N/m (75 dynes/cm) or less, exhibited a higher concentration rate.

On the other hand, in Comparative Examples A1 and A5 in which the hole diameter of the porous membrane was more than 10 μm, the injected sample solution was not held in the first container as described above, and thus the sample solution could not be concentrated.

In addition, in Comparative Example A2 in which the hole diameter of the porous membrane was less than 0.05 μm, the sample solution concentrated solution could not be recovered as described above. In addition, in Comparative Examples A3 and A6 which the super absorbent polymer was not provided, the sample solution could not be concentrated.

It has been found that in Examples A1 to A5, the concentration fold ratio is 4-fold with one time of the number of times of centrifugation and in a short time of 7 minutes without the need for reverse spin centrifugation, whereas in Comparative Example A4, two times of the number of times of centrifugation is required, and three times or more of the concentration time is taken for obtaining the same level of the concentration rate.

Regarding the optical densities obtained by using the LAM kit, the densities of Examples A1 to A5 and Comparative Example A4 were about the same level, and about 1/4 of the densities thereof was obtained from the measurement of Comparative Examples A1 and A7. The concentration of Comparative Example A6 was still lower. It has been found that 4-fold of the detection sensitivity can be achieved by the concentration means that can be operated in a

EP 4 212 873 B1

short time by using a kit having the configuration of the present invention.

[B] Case where antigen is influenza virus

[Preparation of sample solution]

A sample solution (an antigen-containable solution) was prepared using a Quick S-Influ A·B "SEIKEN" negative/positive control solution (product number: 322968, manufactured by DENKA SEIKEN Co., Ltd.).
Specifically, the above control solution was subjected to dilution with a phosphate buffered salts (PBS) buffer containing 0.1% by mass bovine serum albumin (BSA), and sample solutions (antigen-containable solutions) with the respective concentrations were prepared. Here, the concentration (fold) is "control solution/(control solution + buffer), and for example, in a case where the control solution is diluted 100-fold with a buffer, the concentration is 0.01-fold.

[Production of immunochromatographic kit for detecting influenza virus]

An immunochromatographic kit (a detection device) for detecting an influenza virus, for detecting an influenza virus as a test substance (a high-molecular-weight molecule contained in a biological fluid), was produced as follows.

(1-1) Production of anti-influenza A type antibody-modified gold colloid as labeling substance modified with first substance capable of binding to test substance

1 mL of a 50 mmol/L $KH_2PO_4$ buffer (pH 7.5) was added to 9 mL of a solution containing a gold colloid having a diameter of 50 nm (product number: EM. GC50, manufactured by BBI Solutions Inc.) to adjust the pH, and then 1 mL of a solution containing 160 μg/mL of an anti-influenza A type monoclonal antibody (Anti-Influenza A SPTN-5 7307, manufactured by Medix Biochemica Inc.) was added thereto, and stirring was carried out for 10 minutes. Then, after allowing to stand for 10 minutes, 550 μL of an aqueous solution containing 1% by mass of polyethylene glycol ((PEG), weight-average molecular weight (Mw.): 20,000, product number: 168-11285, manufactured by FUJIFILM Wako Pure Chemical Corporation) was added thereto, stirring was carried out for 10 minutes, and subsequently 1.1 mL of an aqueous solution of 10% by mass of bovine serum albumin ((BSA), Fraction V, product number: A-7906, manufactured by Sigma-Aldrich Co., LLC) was added thereto, and stirring was carried out for 10 minutes. This solution was subjected to centrifugal separation for 30 minutes under the conditions of 8,000 × g at 4°C using a centrifugal separator (himac CF16RX, manufactured by Hitachi, Ltd.). The supernatant solution was removed with 1 mL thereof remaining at the bottom of a container, and the gold colloids contained in the 1 mL solution remaining at the bottom of the container were re-dispersed with an ultrasonic washer. Then, they were dispersed in 20 mL of a gold colloid preservative solution (a 20 mmol/L Tris-HCl buffer (pH 8.2), 0.05% PEG (Mw.: 20,000), 150 mmol/L NaCl, 1% BSA), centrifugal separation was carried out again using the same centrifugal separator under the same conditions, the supernatant solution was removed, and after ultrasonic dispersion, they were dispersed in the gold colloid preservative solution to obtain an anti-influenza A type antibody-modified gold colloid (50 nm) solution.

(1-2) Production of anti-influenza A type antibody-modified gold colloid holding pad as label holding pad

The anti-influenza A type antibody-modified gold colloid produced in (1-1) was diluted with water so that a concentration of a Tris-HCl buffer (pH 8.2) was 20 mmol/L, a concentration of PEG (Mw: 20,000) was 0.05% by mass, a concentration of sucrose was 5% by mass, and an optical density of the gold colloid at 520 nm was 0.1 in a case where an optical path length was set to 10 mm, and it was used as a gold colloid coating liquid. 0.8 mL of this coating liquid was uniformly applied onto each glass fiber pad (Glass Fiber Conjugate Pad, manufactured by Millipore Corporation) cut into 12 mm × 300 mm, and drying under reduced pressure was carried out for 24 hours to obtain an anti-influenza A type antibody-modified gold colloid holding pad (a gold colloid holding pad).

(1-3) Production of chromatographic carrier

As an insoluble carrier, a nitrocellulose membrane (having a plastic lining, HiFlow Plus HF135 (capillary flow rate = 135 seconds/cm), manufactured by Millipore Corporation) cut into a size of 60 mm × 300 mm was used, and an examination region, a checking region, and an amplification indicator region were formed on this membrane to produce a chromatographic carrier.
An anti-influenza A type monoclonal antibody (Anti-Influenza A SPTN-5 7307, manufactured by Medix Biochemica Inc.) solution prepared to have a concentration of 1.5 mg/mL was applied in a line shape at a position 15 mm from the downstream side of the short side of 60 mm of the nitrocellulose membrane and used as an examination region.

Further, at a position 11 mm from the downstream side of the short side of 60 mm, an anti-mouse IgG antibody (Anti-mouse IgG (H + L), rabbit F(ab')2, product number: 566-70621, manufactured by FUJIFILM Wako Pure Chemical Corporation) solution prepared to have a confirmation of 0.2 mg/mL was applied in a line shape and used as a checking region. Further, Bromocresol Green (manufactured by FUJIFILM Wako Pure Chemical Corporation) prepared to have a concentration of 30 mmol/L was applied in a line shape at a position 9 mm from the downstream side of the short side of 60 mm and used as an amplification indicator region. After each application, the nitrocellulose membrane was dried at 50°C for 30 minutes in a hot air dryer. After the drying was completed, the nitrogen membrane dried as described above was immersed in a vat containing 500 mL of a borate buffer (pH 8.5) of 50 mmol/L, which contained a blocking solution (0.5% by mass casein (derived from milk, product number: 030-01505, manufactured by FUJIFILM Wako Pure Chemical Corporation)), and allowed to stand for 30 minutes as it was. Then, the nitrocellulose membrane was taken out, immersed in a 500 mL of a washing and stabilizing solution (a 50 mmol/L Tris-HCl (pH 7.5) buffer containing 0.5% by mass sucrose and 0.05% by mass sodium cholate) prepared in another vat, and allowed to stand as it was for 30 minutes. Then, the nitrocellulose membrane was taken out from the solution and dried in an environment of 25°C for 24 hours.

The portion in which the anti-influenza A type antibody is immobilized corresponds to the examination region containing the second substance that binds to a test substance, the portion in which the anti-mouse IgG antibody is immobilized corresponds to the checking region containing a substance that is capable of binding to the first substance, and the portion in which Bromocresol Green is immobilized corresponds to the amplification indicator region containing a substance that reacts with the second amplification solution, which is the reducing agent solution, enclosed in the second pot described below.

(1-4) Production of examination strip

The chromatographic carrier produced in (1-3) was attached to a back pressure-sensitive adhesive sheet (60 mm × 300 mm (manufactured by NIPPN Techno Cluster, Inc.)). Next, a double-sided tape (Nitto Denko Corporation) having a width of 3 mm was fixed at a position 26 mm from the downstream side of the short side of the chromatographic carrier. Then, the gold colloid holding pad was fixed to the chromatographic carrier so that the downstream end of the double-sided tape and the downstream end of the gold colloid holding pad produced in (1-2) overlapped each other. A liquid feeding pad (a glass fiber pad cut into 25 mm × 300 mm (Glass Fiber Conjugate Pad, manufactured by Millipore Corporation)) was attached to the upstream side of the chromatographic carrier so that the liquid feeding pad and the chromatographic carrier overlapped with each other by 7 mm. The member produced in this way was cut to have a width of 5 mm with a guillotine type cutter (CM4000, manufactured by NIPPN Techno Cluster, Inc.) in parallel with a direction perpendicular to the long side of 300 mm, thereby producing sixty examination strips (however, the absorption pad was not included).

(1-5) Preparation of amplification solution

(1-5-1) Preparation of amplification solution (reducing agent solution) to be enclosed in second pot

23.6 mL of an aqueous solution of 1 mol/L iron nitrate, which was produced by dissolving iron (III) nitrate nonahydrate (manufactured by FUJIFILM Wako Pure Chemical Corporation, 095-00995) in water, and 13.1 g of citric acid (manufactured by FUJIFILM Wako Pure Chemical Corporation, 038-06925) were dissolved in 290 g of water. After all of the substances were dissolved, 36 mL of a nitric acid (10% by weight) solution was added thereto while stirring with a stirrer, and 60.8 g of ammonium iron (II) sulfate hexahydrate (manufactured by FUJIFILM Wako Pure Chemical Corporation, 091-00855) was added thereto. The solution prepared in this way was used as a reducing agent solution which was the second amplification solution to be enclosed in the second pot.

(1-5-2) Preparation of amplification solution (silver ion solution) to be enclosed in first pot

8 mL of a silver nitrate solution (including 10 g of silver nitrate) and 24 mL of an aqueous solution of 1 mol/L iron nitrate were added to 66 g of water. Further, this solution was mixed with a solution obtained by dissolving 5.9 mL of nitric acid (10% by weight), 0.1 g of dodecyl amine (manufactured by FUJIFILM Wako Pure Chemical Corporation, 123-00246), and 0.1 g of a surfactant $C_{12}H_{25}$-$C_6H_4$-O-$(CH_2CH_2O)_{50}$H in 47.6 g of water in advance, and the resultant solution was used as a silver ion solution which was the first amplification solution to be enclosed in the first pot.

(1-6) Production of absorption pad

Sixty sheets of glass fiber pads (glass filter paper, manufactured by Advantech Co., Ltd.) cut into 12 mm × 10 mm were prepared and used as absorption pads.

(1-7) Production of parts of immunochromatographic kit

The lower case 20, the upper case 10, the intermediate member 30, and the first pot 40, as well as the second pot 45, which constitute the immunochromatographic kit 100 as illustrated in Fig. 5, Fig. 6, and the like, were each formed by injection molding using polypropylene as a material. The upper case was produced by injection molding using, as a material, polypropylene containing 50% by mass of TAFTHREN (registered trade name), which is an olefin-based elastomer manufactured by Sumitomo Chemical Co., Ltd.

It is noted that the upper case 10 includes two deformable portions (the first convex deformation part and the second convex deformation part), and these two deformable portions do not have a portion separated from the upper case 10, which were produced by injection molding as part of the upper case 10 in the entire boundary part.

It is noted that the upper case is configured such that the first convex deformation part 12 illustrated in Fig. 5, Fig. 6, and the like has two protruding parts, and the second convex deformation part 14 has one protruding part. The bending elastic moduli of the materials of the upper case and the lower case were 90 (MPa) and 700 (MPa), respectively.

(1-8) Production of immunochromatographic kit

The lower case 20, the examination strip 1 produced in (1-4), and the absorption pad 6 produced in (1-6) were fixed as illustrated in Fig. 5 and Fig. 6. Next, the first pot 40 and the second pot 45 were respectively filled with the first amplification solution 41 to be enclosed in the first pot 40 and the second amplification solution 46 to be enclosed in the second pot 45, which were prepared in (1-5-2) and (1-5-1), the second pot 45 was sealed with an aluminum foil as the sheet member 48, the first pot 40 was sealed with an aluminum foil as the sheet member 43, and as illustrated in Fig. 5 and Fig. 6, the second pot 45 was mounted on the lower case 20 with the sheet member 48 facing up, and the first pot 40 was mounted on the intermediate member 30 with the sheet member 43 facing down. Then, in a state where the upper case 10 and the lower case 20 were fitted to each other so that the outer circumferences thereof come into contact with each other, the contact part between the upper case and the lower case was joined by ultrasonic welding. At this time, it was confirmed that the welded parts were uniformly welded at all the parts in a sealed state. In this way, an immunochromatographic kit for detecting an influenza virus was produced.

[Example B1]

[Production of concentration device]

A concentration device was produced according to the same procedure as in Example A1.

[Concentration of sample solution]

Using the obtained concentration device, the above-described sample solution (the sample solution containing the influenza virus) was concentrated according to the same procedure as in Example A1 described above. Specifically, the above-described sample solution (the sample solution containing an influenza virus) was concentrated according to the same procedure as in Example A1, except that in the sample solution injection step, 700 μL of the sample solution (the sample solution containing the influenza virus) was used instead of 700 μL of the sample solution (the sample solution containing LAM).

The amount of the obtained sample solution concentrated solution was 100 μL. The concentration time (the time (minutes) taken from the sample solution injection step to the recovery step) was 7 minutes.

[Detection of influenza virus]

The obtained sample solution concentrated solution was subjected to the detection of the influenza virus as follows.

(2-1) Dropwise addition

The obtained sample solution concentrated solution (40 μL) was added dropwise to the gold colloid holding pad of the immunochromatographic kit for detecting an influenza virus, which was produced as described above. As a result, gold particle composite bodies, which are composite bodies of the influenza A type virus in the sample solution and the gold colloid particles (modified gold particles) modified with the influenza A type monoclonal antibody in the gold colloid holding pad, were formed. In this state, the gold particle composite bodies were spread toward the downstream side of the nitrocellulose membrane.

(2-2) Spreading of amplification solution (reducing agent solution) to be enclosed in second pot

Immediately after the dropwise addition of the sample solution concentrated solution in (2-1), the second convex deformation part 14 was depressed to break the aluminum foil which was the sheet member 48 that sealed the second amplification solution 46 enclosed in the second pot 45, and the liquid feeding pad 4 was immersed in the second pot 45 to supply the second amplification solution 46 to the insoluble carrier 2 by utilizing capillary action.

(2-3) Silver amplification

After the amplification indicator region $L_3$ was discolored from green to orange, the first convex deformation part 12 (the first convex deformation part 114 in Example 2) was depressed to move the first pot 40 toward the breaking part 34 of the pot accommodating part 32 of the intermediate member 30, thereby pushing and breaking the aluminum foil which was the sheet member 43 that sealed the first pot 40, with the breaking part 34, and the silver ion solution, which was the first amplification solution 41, was supplied to the insoluble carrier 2 from the opening portion of the intermediate member 30 to carry out the silver amplification reaction. The silver amplification reaction is completed in several tens of seconds.

(2-4) Evaluation of concentration rate

The coloration of the examination region $L_1$ was visually checked, and as a result of investigating the lowest concentration (C1) in which the coloration was confirmed, it was 0.01-fold. In addition, the detection of the influenza virus was carried out in the same manner without concentrating the sample solution, and as a result of investigating the lowest concentration (C0) in which coloration was confirmed, it was 0.04-fold. Here, since the inverse of the concentration (C1) is approximately proportional to the concentration rate, the inverse of C1 divided by the inverse of C0 was calculated as the concentration rate. The results are shown in Table 2. In a case where the concentration rate is more than 1, it means that the sample solution has been concentrated. It is preferable that the concentration rate is high.

[Example B2]

A concentration device was produced according to the same procedure as in Example A1, except that SAP Spheres (manufactured by M2 Polymer Technologies Inc., swelling ratio: 20 g/g) were used as the SAP particles.
Using the obtained concentration device, the concentration of the sample solution and the detection of the influenza virus were carried out in the same manner as in Example B1. The lowest concentration (C1) in which coloration was confirmed was 0.01-fold. The concentration rate is shown in Table 2.
The amount of the obtained sample solution concentrated solution was 40 μL, and the concentration time was 4 minutes.

[Example B3]

A concentration device was produced according to the same procedure as in Example A3.
Using the obtained concentration device, the concentration of the sample solution and the detection of the influenza virus were carried out in the same manner as in Example B1. The lowest concentration (C1) in which coloration was confirmed was 0.01-fold. The concentration rate is shown in Table 2.

[Example B4]

A concentration device was produced according to the same procedure as in Example A4.
Using the obtained concentration device, the concentration of the sample solution and the detection of the influenza virus were carried out in the same manner as in Example B1. The lowest concentration (C1) in which coloration was confirmed was 0.01-fold. The concentration rate is shown in Table 2.

[Example B5]

A concentration device was produced according to the same procedure as in Example A5.
Using the obtained concentration device, the concentration of the sample solution and the detection of the influenza virus were carried out in the same manner as in Example B1. The lowest concentration (C1) in which coloration was confirmed was 0.012-fold. The concentration rate is shown in Table 2.

[Example B6]

A concentration device was produced according to the same procedure as in Example A6.

Using the obtained concentration device, the concentration of the sample solution and the detection of the influenza virus were carried out in the same manner as in Example B1. The lowest concentration (C1) in which coloration was confirmed was 0.01-fold. The concentration rate is shown in Table 2.

[Example B7]

A concentration device was produced according to the same procedure as in Example A1, except that particles (particle diameter: 0.05 mm, swelling ratio: 600 g/g, water absorption rate: 20 g/min) obtained by grading commercially available SAP (super absorbent polymer) particles (model number: 197-12451, manufactured by Fujifilm Wako Pure Chemical Corporation) was used as the SAP particles.

Using the obtained concentration device, the concentration of the sample solution and the detection of the influenza virus were carried out in the same manner as in Example B1. The lowest concentration (C1) in which coloration was confirmed was 0.02-fold. The concentration rate is shown in Table 2.

[Example B8]

A concentration device was produced according to the same procedure as in Example A1, except that particles (particle diameter: 2.5 mm, swelling ratio: 13 g/g, water absorption rate: 0.5 g/min) obtained by grading commercially available SAP (super absorbent polymer) particles (SAP Sphere 2.5 mm, manufactured by M2 Polymer Technologies Inc.) was used as the SAP particles.

Using the obtained concentration device, the concentration of the sample solution and the detection of the influenza virus were carried out in the same manner as in Example B1. The lowest concentration (C1) in which coloration was confirmed was 0.01-fold. The concentration rate is shown in Table 2.

[Comparative Example B1]

A concentration device was produced according to the same procedure as in Comparative Example A1.

As a result of making an attempt to carry out the concentration of the sample solution in the same manner as in Example B1, by using the obtained concentration device, the sample solution injected into the first container was entirely discharged from the discharge unit, without being held in the first container, and recovered in the second container. The recovered sample solution was 700 $\mu$L. The sample solution recovered in the second container was subjected to the detection of the influenza virus in the same manner as in Example B1. The lowest concentration (C1) in which coloration was confirmed was 0.04-fold. The concentration rate is shown in Table 2.

[Comparative Example B2]

A concentration device was produced according to the same procedure as in Comparative Example A2.

As a result of making an attempt to carry out the concentration of the sample solution in the same manner as in Example B1, by using the obtained concentration device, the sample solution concentrated solution was not discharged from the porous membrane by centrifugation for a short time in the discharge step, and thus the sample solution concentrated solution could not be recovered. As a result, the influenza virus was not detected.

[Comparative Example B3]

Instead of the concentration device of Example B1, a mini-centrifugal filtration filter (VIVACLEAR MINI 0.8 $\mu$m, the material of the porous membrane: polyether sulfone (PES), the hole diameter of the porous membrane: 0.8 $\mu$m, manufactured by Sartorius AG) (a filter without having SAP particles) was used to carry out the sample solution injection step, the discharge step, and the recovery step in the same manner as in Example B1. The entire sample solution injected into the first container was recovered in the second container. The amount of the recovered sample solution was 700 $\mu$L. The recovered sample solution recovered was subjected to the detection of the influenza virus in the same manner as in Example B1. The lowest concentration (C1) in which coloration was confirmed was 0.04-fold. The concentration rate is shown in Table 2.

[Comparative Example B4]

The above-described sample solution was concentrated using Amicon Ultra (3k (3k is 3 KDa and a fractional molecular weight)) (manufactured by Merck KGaA; UFC5003BK). As illustrated in Fig. 13, the product consists of a filtration membrane unit and a liquid-passable recovery container. It is a product that recovers a concentrated solution from the filtration membrane unit by carrying out centrifugation in a state where the filtration membrane unit is inserted into the liquid-passable recovery container (having no filtration membrane). In a method of recovering a concentrated solution remaining in the filtration membrane unit, a filtration membrane unit is inserted into the liquid-passable recovery container with the direction of insertion into the recovery container being opposite to the direction at the time of the first centrifugation, and centrifugation is carried out at a low rotation speed, whereby the concentrated solution can be recovered.

The filtration membrane that is used in the filtration membrane unit is manufactured by Millipore Corporation, where a low-adsorption regenerated cellulose membrane is used. It is a membrane presumed to have a hole diameter of 0.05 $\mu$m or less since the fractional molecular weight is 3KDa although the hole diameter thereof is not described. There are two liquid-passable recovery containers, one of which is a container for recovering or discarding a liquid that has passed through the filtration membrane unit. The other is a container for recovering the remaining concentrated solution in the filtration membrane unit.

700 $\mu$L of the sample solution was placed in a filtration membrane unit of such a product. It was subjected to centrifugation at a rotation speed of 8,000 rpm at 4°C for 20 minutes in a state of being inserted into a liquid-passable recovery container 1. At this time, 370 $\mu$L of the liquid moved to the liquid-passable recovery container side, and a small amount of the liquid remained in the filtration membrane unit. The liquid-passable recovery container was replaced with a new container (a liquid-passable recovery container 2), and an ultrafiltration unit was inserted into the liquid-passable recovery container with the direction of insertion into the recovery container being opposite to the direction at the time of the first centrifugation and subjected to centrifugation at 2,000 rpm for 2 minutes (referred to as reverse spin centrifugation). As a result, the amount of the obtained sample solution concentrated solution was 130 $\mu$L. At this time, the time required for concentration was 26 minutes. The obtained sample solution concentrated solution was subjected to the detection of the influenza virus in the same manner as in Example B1. The lowest concentration (C1) in which coloration was confirmed was 0.015-fold. The concentration rate is shown in Table 2.

[Comparative Example B5]

A concentration device was produced according to the same procedure as in Comparative Example A5.

As a result of making an attempt to carry out the concentration of the sample solution in the same manner as in Example B1, by using the obtained concentration device, the sample solution injected into the first container was entirely discharged from the discharge unit, without being held in the first container, and recovered in the second container. The recovered sample solution was 700 $\mu$L. The sample solution recovered in the second container was subjected to the detection of the influenza virus in the same manner as in Example B1. The lowest concentration (C1) in which coloration was confirmed was 0.04-fold. The concentration rate is shown in Table 2.

[Table 2]

| | Example B1 | Example B2 | Example B3 | Example B4 | Example B5 | Example B6 | Example B7 | Example B8 | Comparative Example B1 | Comparative Example B2 | Comparative Example B3 | Comparative Example B4 | Comparative Example B5 |
|---|---|---|---|---|---|---|---|---|---|---|---|---|---|
| SAP swelling ratio [g/g] | 30 | 20 | 30 | 30 | 30 | 30 | 600 | 13 | 30 | 30 | - | - | 30 |
| Porous membrane material | PES | PES | CA | GF | | | PES | PES | GF | CA | PES | CA | PP |
| Pore diameter [μm] | 0.8 | 0.8 | 0.2 | 0.45 | 0.05 | 0.05 | 0.8 | 0.8 | 100 | <0.01 | 0.8 | | 50 |
| Surface energy [10⁻³N/m (dyne/cm)] | 46 | 46 | 50 | 73 | 35 | 23 | 46 | 46 | 73 | 50 | 46 | 50 | 30 |
| Number of times of centrifugation | 1 | 1 | 1 | 1 | 1 | 1 | 1 | 1 | - | 1 | 1 | 2 | - |
| Concentration time [minute] | 7 | 7 | 7 | 7 | 7 | 7 | 7 | 7 | - | - | 7 | 16 | - |
| Sample concentration in which minimum detection sensitivity is obtained [fold] | 0.01 | 0.01 | 0.01 | 0.01 | 0.012 | 0.015 | 0.02 | 0.01 | 0.04 | - | 0.04 | 0.015 | 0.04 |
| Concentration rate | 4 | 4 | 4 | 4 | 3.3 | 2.7 | 2 | 4 | 1 | - | 1 | 2.7 | 1 |

In Table 2, the "SAP swelling ratio" indicates the swelling ratio [g/g] of the super absorbent polymer used. The method of measuring the swelling ratio is as described above.

In Table 2, the "porous membrane material" indicates the material of the porous membrane used, PES indicates polyether sulfone, CA indicates cellulose acetate, GF indicates glass fiber, hydrophilic PTFE indicates hydrophilic polytetrafluoroethylene, hydrophobic PTFE indicates hydrophobic polytetrafluoroethylene, and PP indicates polypropylene.

In Table 2, the "hole diameter" indicates the hole diameter [μm] of the porous membrane used.

In Table 2, the "surface energy" indicates the surface energy [N/m (dynes/cm)] of the material of the porous membrane used. The method of measuring the surface energy is as described above.

In Table 2, the "concentration time" indicates the time (minutes) taken from the sample solution injection step to the recovery step. Practically, the concentration time is preferably 20 minutes or less.

In Table 2, "Sample concentration in which minimum detection sensitivity is obtained" indicates the above-described "lowest concentration in which coloration was confirmed".

As can be seen from Table 2, in a case where Examples B1 to B8, which are the concentration devices according to the embodiment of the present invention, were used, the sample solution could be concentrated in a short time (20 minutes or less).

From the comparison among Examples B1 to B6 (the comparison among the aspects in which only the porous membrane is different), Examples B1 to B5, in which the porous membrane consisted of a material having a surface energy of $24.10^{-3}$ N/m (24 dynes/cm) or more and $75.10^{-3}$ N/m (75 dynes/cm) or less, exhibited a higher concentration rate. Among them, Examples B1 to B4, in which the porous membrane consisted of a material having a surface energy $40.10^{-3}$ N/m (40 dynes/cm) or more and $75.10^{-3}$ N/m (75 dynes/cm) or less, exhibited a higher concentration rate.

In addition, from the comparison among Examples B1, B7, and B8 (the comparison among aspects in which only the super absorbent polymer is different), it was seen that Examples B1 and B8 in which the swelling ratio of the super absorbent polymer is 500 g/g or less exhibit high concentration rate.

On the other hand, in Comparative Examples B1 and B5 in which the hole diameter of the porous membrane was more than 10 μm, the injected sample solution was not held in the first container as described above, and thus the sample solution could not be concentrated. In addition, in Comparative Example B2 in which the hole diameter of the porous membrane was less than 0.05 μm, the sample solution concentrated solution could not be recovered as described above. In addition, in Comparative Example B3 which the super absorbent polymer was not provided, the sample solution could not be concentrated.

Explanation of References

1: examination strip
2: insoluble carrier
3: label holding pad
4: liquid feeding pad
6: absorption pad
7: back pressure-sensitive adhesive sheet
9: housing case
10: upper case
12: first convex deformation part
12a: top of first convex deformation part
12b: protruding part of first convex deformation part
12c: slope of first convex deformation part
14: second convex deformation part
14a: top of second convex deformation part
14b: protruding part of second convex deformation part
16: opening pore for dropwise addition of specimen solution
18: observation window
20: lower case
21: insoluble carrier accommodating part
22: absorption pad accommodating part
24: second pot accommodating part
30: intermediate member
32: first pot accommodating part
34: breaking part
35: flow channel forming part
36: back surface of flow channel forming part 35
40: first pot for first amplification solution
41: first amplification solution
42: pot container
43: sheet member
45: second pot for second amplification solution
46: second amplification solution
47: pot container
48: sheet member
100: immunochromatographic kit
114: convex deformation part
114a: top of convex deformation part 114
114b: protruding part of convex deformation part 114
200, 201, 202, 203, 204: concentration device
200a: mini-centrifugal filtration filter
210: first container
211: opening portion
212: discharge unit
220: second container
221: opening portion
230: super absorbent polymer
232: swollen super absorbent polymer
240: sample solution
242: sample solution concentrated solution
300: nitrocellulose membrane
301: gold colloid holding pad
302: test line
303: control line
304: coloring reagent immobilization line

**EP 4 212 873 B1**

**Claims**

1. A concentration device (200) for concentrating a sample solution (240) which is an aqueous solution containing a high-molecular-weight molecule contained in a biological fluid, the concentration device (200) comprising:

   a first container (210) containing a super absorbent polymer (230), and
   a second container (220),
   wherein a part of the first container (210) is constituted of a discharge unit (212) consisting of a porous membrane,
   the super absorbent polymer (230) is configured to absorb a solution contained in the sample solution (240) injected into the first container (210) to generate, in the first container (210), a sample solution concentrated solution (242) which is a concentrated solution of the sample solution (240), and
   the second container (220) is configured to recover the sample solution concentrated solution (242) discharged from the discharge unit (212), and wherein the device is **characterised in that** the porous membrane has a pore diameter of 0.05 $\mu$m or more and 10 $\mu$m or less and the porous membrane consists of a material having a surface energy of $24.10^{-3}$ N/m (24 dynes/cm) or more and $75.10^{-3}$ N/m (75 dynes/cmm) or less.

2. The concentration device according to claim 1,
   wherein a material of the porous membrane is at least one selected from the group consisting of cellulose acetate, polyether sulfone, and glass fiber.

3. The concentration device according to claim 1 or 2,
   wherein the porous membrane is consisting of a material having a surface energy of $40.10^{-3}$ N/m (40 dynes/cm) or more and $75.10^{-3}$ N/m (75 dynes/cm) or less.

4. The concentration device according to any one of claims 1 to 3,
   wherein a swelling ratio of the super absorbent polymer (230) is 20 g/g or more and less than 800 g/g.

5. The concentration device according to any one of claims 1 to 4,
   wherein the super absorbent polymer (230) is a polyacrylic acid-based, polyacrylamide-based, cellulose-based, or polyethylene oxide-based polymer.

6. The concentration device according to any one of claims 1 to 5,
   wherein the high-molecular-weight molecule contained in the biological fluid is an antigen.

7. The concentration device according to any one of claims 1 to 6,
   wherein the sample solution (240) is urine.

8. The concentration device according to claim 7,
   wherein a concentration of urea in the sample solution concentrated solution (242) is 5-fold or less with respect to the concentration of the urea in the sample solution (240).

9. A sample solution concentration method in which a sample solution (240) which is an aqueous solution containing a high-molecular-weight molecule contained in a biological fluid is concentrated using the concentration device (200) according to any one of claims 1 to 8, the sample solution concentration method comprising, in the following order:

   a sample solution injection step of injecting the sample solution (240) into the first container (210);
   a water absorption step in which a solution contained in the sample solution (240) injected into the first container (210) is absorbed by a super absorbent polymer (230) accommodated in the first container (210) to generate, in the first container (210), a sample solution concentrated solution (242), which is a concentration solution of the sample solution (240);
   a discharge step of applying an external force to the sample solution concentrated solution (242) to discharge the sample solution concentrated solution (242) from the discharge unit (212); and
   a recovery step of recovering the sample solution concentrated solution (242) discharged from the discharge unit (212), in the second container (220).

10. The sample solution concentration method according to claim 9, wherein the external force is a centrifugal force.

11. The sample solution concentration method according to claim 10,

37

wherein the centrifugal force is applied under a condition of 4,000 to 10,000 rpm.

12. A sample solution examination method in which a high-molecular-weight molecule contained in a biological fluid in a sample solution (240) which is an aqueous solution containing the high-molecular-weight molecule contained in the biological fluid is detected, the sample solution examination method comprising, in the following order:

   a concentration step of using the sample solution concentration method according to any one of claims 9 to 11 to obtain the sample solution concentrated solution (242); and
   a detection step of detecting the high-molecular-weight molecule contained in the biological fluid in the obtained sample solution concentrated solution (242).

13. The examination method according to claim 12,

   wherein the sample solution (240) is an aqueous solution in which an antigen is containable,
   the concentration step is a step of using the sample solution concentration method according to any one of claims 9 to 11 to concentrate an aqueous solution in which the antigen is containable, to obtain an antigen-concentrated solution, and
   the detection step is a step of detecting the antigen in the antigen-concentrated solution by immunochromatography using an antigen-antibody reaction.

14. An examination kit (100) for detecting a high-molecular-weight molecule contained in a biological fluid in a sample solution (240) which is an aqueous solution containing the high-molecular-weight molecule contained in the biological fluid, the examination kit (100) comprising:

   the concentration device (200) according to any one of claims 1 to 8; and
   a detection device that includes an examination strip (1) having an examination region (L1) for detecting the high-molecular-weight molecule contained in the biological fluid, a first pot (40) and a second pot (45) in which a first amplification solution (41) and a second amplification solution (46) for amplifying an examination signal in the examination region (L1) are enclosed respectively, and a housing case (9) encompassing the examination strip (1), the first pot (40), and the second pot (45).

**Patentansprüche**

1. Konzentrationsvorrichtung (200) zum Konzentrieren einer Probenlösung (240), die eine wässrige Lösung ist, die ein Molekül mit hohem Molekulargewicht, das in einem biologischen Fluid enthalten ist, enthält, wobei die Konzentrationsvorrichtung (200) umfasst:

   einen ersten Behälter (210), der ein superabsorbierendes Polymer (230) enthält, und
   einen zweiten Behälter (220),
   wobei ein Teil des ersten Behälters (210) aus einer Abgabeeinheit (212) besteht, die aus einer porösen Membran besteht,
   das superabsorbierende Polymer (230) so konfiguriert ist, dass es eine in der Probenlösung (240) enthaltene Lösung absorbiert, die in den ersten Behälter (210) eingespritzt wird, um in dem ersten Behälter (210) eine probenlösungskonzentrierte Lösung (242), die eine konzentrierte Lösung der Probenlösung (240) ist, zu erzeugen, und
   der zweite Behälter (220) so konfiguriert ist, dass er die probenlösungskonzentrierte Lösung (242), die aus der Abgabeeinheit (212) abgegeben wird, zurückgewinnt, und wobei die Vorrichtung **dadurch gekennzeichnet ist, dass** die poröse Membran einen Porendurchmesser von 0,05 $\mu$m oder mehr und 10 $\mu$m oder weniger aufweist und die poröse Membran aus einem Material, das eine Oberflächenenergie von $24,10^{-3}$ N/m (24 dyn/cm) oder mehr und $75,10^{-3}$ N/m (75 dyn/cm) oder weniger aufweist, besteht.

2. Konzentrationsvorrichtung nach Anspruch 1,
   wobei ein Material der porösen Membran mindestens eines ist, das aus der Gruppe, die aus Celluloseacetat, Polyethersulfon und Glasfaser besteht, ausgewählt wird.

3. Konzentrationsvorrichtung nach Anspruch 1 oder 2,
   wobei die poröse Membran aus einem Material, das eine Oberflächenenergie von $40,10^{-3}$ N/m (40 dyn/cm) oder mehr

und 75,10$^{-3}$ N/m (75 dyn/cm) oder weniger aufweist, besteht.

4. Konzentrationsvorrichtung nach einem der Ansprüche 1 bis 3,
   wobei ein Quellverhältnis des superabsorbierenden Polymers (230) 20 g/g oder mehr und weniger als 800 g/g beträgt.

5. Konzentrationsvorrichtung nach einem der Ansprüche 1 bis 4,
   wobei das superabsorbierende Polymer (230) ein Polymer auf Polyacrylsäurebasis, Polyacrylamidbasis, Cellulosebasis oder Polyethylenoxidbasis ist.

6. Konzentrationsvorrichtung nach einem der Ansprüche 1 bis 5,
   wobei das Molekül mit hohem Molekulargewicht, das in dem biologischen Fluid enthalten ist, ein Antigen ist.

7. Konzentrationsvorrichtung nach einem der Ansprüche 1 bis 6,
   wobei die Probenlösung (240) Urin ist.

8. Konzentrationsvorrichtung nach Anspruch 7,
   wobei eine Konzentration von Harnstoff in der probenlösungskonzentrierten Lösung (242) in Bezug auf die Konzentration des Harnstoffs in der Probenlösung (240) 5 -fach oder weniger beträgt.

9. Probenlösungs-Konzentrationsverfahren, bei dem eine Probenlösung (240), die eine wässrige Lösung ist, die ein Molekül mit hohem Molekulargewicht, das in einem biologischen Fluid enthalten ist, enthält, unter Verwendung der Konzentrationsvorrichtung (200) nach einem der Ansprüche 1 bis 8 konzentriert wird, wobei das Probenlösungs-Konzentrationsverfahren in der folgenden Reihenfolge umfasst:

   einen Probenlösung-Einspritzschritt des Einspritzens der Probenlösung (240) in den ersten Behälter (210);
   einen Wasserabsorptionsschritt, bei dem eine in der Probenlösung (240) enthaltene Lösung, die in den ersten Behälter (210) eingespritzt wird, von einem superabsorbierenden Polymer (230), das in dem ersten Behälter (210) aufgenommen ist, absorbiert wird, um in dem ersten Behälter (210) eine probenlösungskonzentrierte Lösung (242), die eine Konzentrationslösung der Probenlösung (240) ist, zu erzeugen;
   einen Abgabeschritt des Ausübens einer externen Kraft auf die probenlösungskonzentrierte Lösung (242), um die probenlösungskonzentrierte Lösung (242) aus der Abgabeeinheit (212) abzugeben; und
   einen Rückgewinnungsschritt des Rückgewinnens der probenlösungskonzentrierten Lösung (242), die aus der Abgabeeinheit (212) abgegeben wird, in dem zweiten Behälter (220).

10. Probenlösungs-Konzentrationsverfahren nach Anspruch 9,
    wobei die externe Kraft eine Zentrifugalkraft ist.

11. Probenlösungs-Konzentrationsverfahren nach Anspruch 10,
    wobei die Zentrifugalkraft unter einer Bedingung von 4.000 bis 10.000 U/min ausgeübt wird.

12. Probenlösung-Untersuchungsverfahren, bei dem ein Molekül mit hohem Molekulargewicht, das in einem biologischen Fluid in einer Probenlösung (240) enthalten ist, die eine wässrige Lösung ist, die das Molekül mit hohem Molekulargewicht, das in dem biologischen Fluid enthalten ist, enthält, detektiert wird, wobei das Probenlösung-Untersuchungsverfahren in der folgenden Reihenfolge umfasst:

    einen Konzentrationsschritt des Verwendens des Probenlösungs-Konzentrationsverfahrens nach einem der Ansprüche 9 bis 11, um die probenlösungskonzentrierte Lösung (242) zu erhalten; und
    einen Detektionsschritt des Detektierens des Moleküls mit hohem Molekulargewicht, das in dem biologischen Fluid in der erhaltenen probenlösungskonzentrierten Lösung (242) enthalten ist.

13. Untersuchungsverfahren nach Anspruch 12,

    wobei die Probenlösung (240) eine wässrige Lösung ist, in der ein Antigen enthalten sein kann,
    der Konzentrationsschritt ein Schritt des Verwendens des Probenlösungs-Konzentrationsverfahrens nach einem der Ansprüche 9 bis 11 zum Konzentrieren einer wässrigen Lösung, in der das Antigen enthalten sein kann, um eine antigenkonzentrierte Lösung zu erhalten, ist, und
    der Detektionsschritt ein Schritt des Detektierens des Antigens in der antigenkonzentrierten Lösung durch Immunochromatographie unter Verwendung einer Antigen-Antikörper-Reaktion ist.

**14.** Untersuchungskit (100) zum Detektieren eines Moleküls mit hohem Molekulargewicht, das in einem biologischen Fluid in einer Probenlösung (240) enthalten ist, die eine wässrige Lösung ist, die das Molekül mit hohem Molekulargewicht, das in dem biologischen Fluid enthalten ist, enthält, wobei das Untersuchungskit (100) umfasst:

die Konzentrationsvorrichtung (200) nach einem der Ansprüche 1 bis 8; und
eine Detektionsvorrichtung, die einen Untersuchungsstreifen (1) mit einem Untersuchungsbereich (L1) zum Detektieren des Moleküls mit hohem Molekulargewicht, das in dem biologischen Fluid enthalten ist, einen ersten Topf (40) und einen zweiten Topf (45), in denen eine erste Verstärkungslösung (41) und eine zweite Verstärkungslösung (46) zum Verstärken eines Untersuchungssignals in dem Untersuchungsbereich (L1) entsprechend eingeschlossen sind, und einen Gehäusekasten (9), der den Untersuchungsstreifen (1), den ersten Topf (40) und den zweiten Topf (45) einschließt, enthält.

## Revendications

**1.** Dispositif de concentration (200) pour concentrer une solution d'échantillon (240) qui est une solution aqueuse contenant une molécule de poids moléculaire élevé contenue dans un fluide biologique, le dispositif de concentration (200) comprenant :

un premier récipient (210) contenant un polymère superabsorbant (230), et
un deuxième récipient (220),
dans lequel une partie du premier récipient (210) est constituée d'une unité de décharge (212) consistant en une membrane poreuse,
le polymère superabsorbant (230) est configuré pour absorber une solution contenue dans la solution d'échantillon (240) injectée dans le premier récipient (210) pour générer, dans le premier récipient (210), une solution concentrée de solution d'échantillon (242) qui est une solution concentrée de la solution d'échantillon (240), et
le deuxième récipient (220) est configuré pour récupérer la solution concentrée de solution d'échantillon (242) déchargée par l'unité de décharge (212), et dans lequel le dispositif est **caractérisé en ce que** la membrane poreuse a un diamètre de pore de 0,05 $\mu$m ou plus et 10 $\mu$m ou moins et **en ce que** la membrane poreuse est constituée d'un matériau ayant une énergie de surface de $24 \times 10^{-3}$ N/m (24 dynes/cm) ou plus et de $75 \times 10^{-3}$ N/m (75 dynes/cm) ou moins.

**2.** Dispositif de concentration selon la revendication 1,
dans lequel un matériau de la membrane poreuse est au moins un élément choisi parmi le groupe constitué d'acétate de cellulose, de polyéther sulfone et de fibre de verre.

**3.** Dispositif de concentration selon la revendication 1 ou la revendication 2,
dans lequel la membrane poreuse est constituée d'un matériau ayant une énergie de surface de $40 \times 10^{-3}$ N/m (40 dynes/cm) ou plus et de $75 \times 10^{-3}$ N/m (75 dynes/cm) ou moins.

**4.** Dispositif de concentration selon l'une quelconque des revendications 1 à 3,
dans lequel un taux de gonflement du polymère superabsorbant (230) est de 20 g/g ou plus et inférieur à 800 g/g.

**5.** Dispositif de concentration selon l'une quelconque des revendications 1 à 4,
dans lequel le polymère superabsorbant (230) est un polymère à base d'acide polyacrylique, à base de polyacrylamide, à base de cellulose ou à base d'oxyde de polyéthylène.

**6.** Dispositif de concentration selon l'une quelconque des revendications 1 à 5,
dans lequel la molécule de poids moléculaire élevé contenue dans le fluide biologique est un antigène.

**7.** Dispositif de concentration selon l'une quelconque des revendications 1 à 6,
dans lequel la solution d'échantillon (240) est de l'urine.

**8.** Dispositif de concentration selon la revendication 7,
dans lequel une concentration d'urée dans la solution concentrée de solution d'échantillon (242) est de 5 fois ou moins par rapport à la concentration de l'urée dans la solution d'échantillon (240).

9. Procédé de concentration de solution d'échantillon dans lequel une solution d'échantillon (240) qui est une solution aqueuse contenant une molécule de poids moléculaire élevé contenue dans un fluide biologique est concentrée à l'aide du dispositif de concentration (200) selon l'une quelconque des revendications 1 à 8, le procédé de concentration de solution d'échantillon comprenant, dans l'ordre suivant :

une étape d'injection de solution d'échantillon consistant à injecter la solution d'échantillon (240) dans le premier récipient (210) ;
une étape d'absorption d'eau dans laquelle une solution contenue dans la solution d'échantillon (240) injectée dans le premier récipient (210) est absorbée par un polymère superabsorbant (230) logé dans le premier récipient (210) pour générer, dans le premier récipient (210), une solution concentrée de solution d'échantillon (242), qui est une solution concentrée de la solution d'échantillon (240) ;
une étape de décharge consistant à appliquer une force externe à la solution concentrée de solution d'échantillon (242) pour décharger la solution concentrée de solution d'échantillon (242) de l'unité de décharge (212) ; et
une étape de récupération consistant à récupérer la solution concentrée de solution d'échantillon (242) déchargée de l'unité de décharge (212), dans le deuxième récipient (220).

10. Procédé de concentration de solution d'échantillon selon la revendication 9, dans lequel la force externe est une force centrifuge.

11. Procédé de concentration de solution d'échantillon selon la revendication 10,
dans lequel la force centrifuge est appliquée dans une condition de 4 000 à 10 000 tr/min.

12. Procédé d'examen de solution d'échantillon dans lequel une molécule de poids moléculaire élevé contenue dans un fluide biologique dans une solution d'échantillon (240) qui est une solution aqueuse contenant la molécule de poids moléculaire élevé contenue dans le fluide biologique est détectée, le procédé d'examen de solution d'échantillon comprenant, dans l'ordre suivant :

une étape de concentration consistant à utiliser le procédé de concentration de solution d'échantillon selon l'une quelconque des revendications 9 à 11 pour obtenir la solution concentrée de solution d'échantillon (242) ; et
une étape de détection consistant à détecter la molécule de poids moléculaire élevé contenue dans le fluide biologique dans la solution concentrée de solution d'échantillon (242) obtenue.

13. Procédé d'examen selon la revendication 12,

dans lequel la solution d'échantillon (240) est une solution aqueuse dans laquelle un antigène peut être contenu,
l'étape de concentration est une étape consistant à utiliser le procédé de concentration de solution d'échantillon selon l'une quelconque des revendications 9 à 11 pour concentrer une solution aqueuse dans laquelle l'antigène peut être contenu, afin d'obtenir une solution concentrée en antigène, et
l'étape de détection est une étape consistant à détecter l'antigène dans la solution concentrée en antigène par immunochromatographie en utilisant une réaction antigène-anticorps.

14. Kit d'examen (100) pour détecter une molécule de poids moléculaire élevé contenue dans un fluide biologique dans une solution d'échantillon (240) qui est une solution aqueuse contenant la molécule de poids moléculaire élevé contenue dans le fluide biologique, le kit d'examen (100) comprenant :

le dispositif de concentration (200) selon l'une quelconque des revendications 1 à 8 ; et
un dispositif de détection qui inclut une bandelette d'examen (1) ayant une région d'examen (L1) pour détecter la molécule de poids moléculaire élevé contenue dans le fluide biologique, un premier godet (40) et un deuxième godet (45) dans lesquels sont respectivement renfermées une première solution d'amplification (41) et une deuxième solution d'amplification (46) pour amplifier un signal d'examen dans la région d'examen (L1), et un boîtier de logement (9) englobant la bandelette d'examen (1), le premier godet (40) et le deuxième godet (45).

FIG. 1

FIG. 2

FIG. 3A

FIG. 3B

## FIG. 3C

## FIG. 3D

# FIG. 4

300

304 303 302        301

DOWNSTREAM        UPPERTREAM

# FIG. 5

12

18

16

100

14

9
10  20

## FIG. 6

# FIG. 7

DOWNSTREAM ←————————————————————————————→ UPPERTREAM

EP 4 212 873 B1

# FIG. 8

DOWNSTREAM

# FIG. 9

A

B

# FIG. 10

DOWNSTREAM

FIG. 11

A

B

FIG. 12

A

B

# FIG. 13

LIQUID-PASSABLE RECOVERY CONTAINER 1

LIQUID-PASSABLE RECOVERY CONTAINER 2

FILTRATION MEMBRANE UNIT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4355339 A
- JP H4355339 A
- WO 2020108390 A
- JP 2013527225 A
- WO 2017139153A A
- WO 2013129634 A
- JP 5728453 B
- US 6020117 A
- JP 2009150869 A

### Non-patent literature cited in the description

- **YASUAKI KITAZAKI et al.** *Journal of the Adhesion Society of Japan*, 1972, vol. 8 (3), 131-141